(19) Europäisches Patentamt
European Patent Office
Office européen des brevets

(11) Veröffentlichungsnummer: **0 001 104**
**B1**

(12) **EUROPÄISCHE PATENTSCHRIFT**

(45) Veröffentlichungstag der Patentschrift: **20.05.81**

(51) Int. Cl.$^3$: **A 61 K 35/18**

(21) Anmeldenummer: **78100816.4**

(22) Anmeldetag: **04.09.78**

(54) **Modifizierte intakte Erythrozyten, sie enthaltendes Blut bzw. Blutkonserven, und Verfahren zu deren Herstellung.**

(30) Priorität: **06.09.77 DE 2740053**
**11.05.78 DE 2820603**

(43) Veröffentlichungstag der Anmeldung:
**21.03.79 Patentblatt 79/6**

(45) Bekanntmachung des Hinweises auf die Patenterteilung:
**20.05.81 Patentblatt 81/20**

(84) Benannte Vertragsstaaten:
**BE CH DE FR GB LU NL SE**

(56) Entgegenhaltungen:
DE - A - 2 249 552
CHEMICAL ABSTRACTS, vol. 75, 15114n 1—21 (1971)
NATURE, vol. 252, 163—166 (1974)

(73) Patentinhaber: **Studiengesellschaft Kohle mbH**
**Kaiser-Wilhelm-Platz 1**
**D-4330 Mülheim/Ruhr (DE)**

(73) Patentinhaber: **Gersonde, Klaus, Prof. Dr.**
**Preussweg 69**
**D-5100 Aachen (DE)**

(72) Erfinder: **Nicolau, Yves-Claude, Prof. Dr.**
**Leonard-Stinnes-Strasse 48**
**D-4330 Mülheim-Ruhr (DE)**
Erfinder: **Gersonde, Klaus, Prof. Dr.**
**Preusweg 69**
**D-5100 Aachen (DE)**

(74) Vertreter: **von Kreisler, Alek, Dipl.-Chem. et al,**
**Deichmannhaus am Hauptbahnhof**
**D-5000 Köln 1 (DE)**

EP 0 001 104 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Modifizierte intakte Erythrozyten, sie enthaltendes Blut bzw. Blutkonserven, und Verfahren zu deren Herstellung

Eine der Hauptaufgaben der Erythrozyten ist der Transport von molekularem Sauerstoff von der Lunge zu den peripheren Geweben. Die Erythrozyten enthalten in hoher Konzentration Hämoglobin (Hb) (30 pg pro Zelle = 35,5 g/100 ml Zellen), das mit $O_2$ ein reversibles Addukt bildet. Der Sauerstoffpartialdruck in der Lunge beträgt etwa 100 Torr und im Kapillarsystem etwa 70 Torr, gegen den $O_2$ von dem mit Sauerstoff beladenen Hämoglobin dissoziiert werden muß. Unter physiologischen Bedingungen können nur etwa 25% des mit Sauerstoff beladenen Hämoglobins von Sauerstoff entladen werden. Etwa 75% werden mit dem venösen Blut zur Lunge zurückgeführt. Der größere Teil des Hb—$O_2$-Addukts wird somit nicht für den $O_2$-Transport ausgenutzt.

Wechselwirkungen des Hämoglobins mit allosterischen Effektoren ermöglichen eine Anpassung an das physiologische Erfordernis der maximalen Sauerstoffabgabe aus dem Hb—$O_2$-Addukt bei gleichzeitiger Aufrechterhaltung des höchstmöglichen Sauerstoffpartialdrucks im Kapillarsystem.

Die allosterischen Eigenschaften von Hämoglobin sind für die Sigmoid-Form der Sauerstoffbindungskurve verantwortlich. Sein Halbsättigungsdruck ($P_{O_{2(1/2)}}$1) ist ein Maßstab für die $O_2$-Affinität. Die Steigung der sigmoidförmigen Kurve im Bereich von 40 bis 60% Sättigung stell den Grad des Zusammenwirkens (cooperativity) der vier $O_2$-Bindungsssstellen im Hb dar (Hill-Koeffizient: n = 2,8 bis 3,2). Ein Anstieg des Hill-Koeffizienten, d.h. eine steilere Neigung der sigmoidförmigen Bindungskurve oder eine Verschiebung der gesamten Bindungskurve zu höherem $O_2$-Partialdruck (sog. "Rechtsverschiebung") würde zu erleichterter $O_2$-Abgabe im Kapillarsystem und zu verbesserter Sauerstoffversorgung der umgebenden Gewebe führen. Eine "Rechtsverschiebung" wird in vivo tatsächlich durch Erhöhung der 2,3-Bisphosphoglyceratkonzentration in den Erythrozyten und seine Bindung an Hämoglobin erreicht. Durch Bindung von 2,3-Bisphosphoglycerat an Hb wird die $O_2$-Affinität herabgesetzt und der $O_2$-Halbsättigungsdruck erhöht. 2,3-Bisphosphoglycerat, das im Glykolyse-Bypass in Erythrozyten gebildet wird, bewirkt langsame Anpassung an $O_2$-Mangel.

2,3-Bisphosphoglycerat erhöht den Halbsättigungsdruck von isoliertem (stripped) Hämoglobin bei pH 7,4 von $P_{O_{2(1/2)}}$ = 9,3 Torr (37°C) und 4,3 Torr (25°C) auf $P_{O_{2(1/2)}}$ = 23,7 Torr (37°C) bzw. 12,0 Torr (25°C) (K. Imai und T. Yonetani, J. Biol. Chem. 250 (1975) 1093—1098). Eine erheblich größere Verminderung der $O_2$-Affinität, d.h. eine Steigerung des $O_2$-Halbsättigungsdrucks, wurde für isoliertes Hämoglobin durch Bindung von Inosithexaphosphat (Phytinsäure, IHP), das aus Pflanzengeweben isoliert worden ist, erreicht (K. Ruckpaul und Mitarbeiter, Biochim. Biophys. Acta 236 (1971) 211—221). Die Bindung von IHP an Hämoglobin erhöht den $O_2$-Halbsättigungsdruck auf $P_{O_{2(1/2)}}$ = 96,4 Torr (37°C) bzw. $P_{O_{2(1/2)}}$ = 48,4 Torr (25°C). IHP kann-ebenso wie 2,3-Bisphosphoglycerat und andere Polyphosphate die Erythrozytenmembran nicht durchdringen.

Lipid-Vesikel spiegeln gewisse Eigenschaften der Zellmembranen wider. Sie vermögen in die Zellen entweder durch Fusion mit der Zellplasmamembran oder durch Endozytose einzudringen (D. Papahadjopoulos und Mitarbeiter, Nature 252 (1976) 163—165). Die Fusion spielt eine wichtige Rolle bei zahlreichen Membranprozessen, beispielsweise bei der synaptischen übertragung, bei der Sekretion, bei der Bildung der Plasmamembran (Assembly) und bei der Infektion mit Membranviren. Die Fusion der Zellen mit Lipid-Vesikeln wurde sowohl in Zellkulturen als auch bei Erythrozyten-"Ghosts" (Erythrozyten-Membranen ohne Zellinhalt) beobachtet.

Durch Behandlung von ML-Zellen (Zellstamm-Lymphom der Maus) mit Vesikeln, die IgG enthielten, mit hohem Neutralisationstiter gegen Coxsackie-Virus A—2 L wurden die Zellen gegen anschließende Infizierung mit diesem Virus geschützt. Gregoriadis und Buckland (Nature 235 (1973) 252—253) verwendeten Lipid-Vesikel, um Invertase in Makrophagen der Maus, die einen Invertase-Defekt aufwiesen, einzubauen. In-vivo-Versuche führten jedoch zu keinen eindeutigen Ergebnissen, da die Lipid-Vesikel unterschiedslos in alle Zellen inkorporiert oder in der Leber schnell abgebaut werden.

Die Verwendung von Lipid-Vesikeln für den Einbau von allosterischen Effektoren oder anderen Substanzen in Erythrozyten ist bisher nicht beschrieben worden. Zahlreiche Versuche haben ergeben, daß Lipid-Vesikel Tieren gefahrlos injiziert werden können.

Während der Einfluss von IHP auf die Struktur und Funktion von isoliertem Hb eingehend untersucht worden ist, gibt es keine Kenntnis über die Wechselwirkung von IHP mit Hämoglobin in intakten Erythrozyten, da es bisher nicht möglich war, IHP in intakte Erythrozyten einzuführen.

In C.A. Col. 75 (1971) S. 15 123, Referat 15 114, die inhaltlich identisch mit Biochim. Biophys. Acta 236 (1971) S. 211—221 ist, wird erstmalig gezeigt, daß IHP an freies, in wässrigem Medium gelöstes Hämoglobin gebunden wird und den $O_2$-Halbsättigungsdruck erhöht, bzw. die $O_2$-Affinität herabsetzt. Untersuchungen der IHP-Bindung an freies Hb sind in der Literatur sehr zahlreich. Hingegen ist bis heute nichts in der Literatur über die Wirkung von Inositolhexaphosphat in intakten menschlichen roten Blutzellen mitgeteilt, da dieser Effektor nicht in den roten Blutzellen gebildet wird, durch die Erythrozyten-Membran nicht eindringen noch mit Hilfe der bisher bekannten Techniken in ausreichenden Mengen durch die Erythrozyten-Membran eingeschleust werden kann.

In "Nature" Vol 252, S. 163—166 (1974) wird gezeigt, daß Lipidvesikel in Membranen von Erythrozyten-Ghosts (sog. "Geisterzellen", d.h. rote Blutzellen ohne Inhalt) inkorporiert werden. Der

Einbau von radioaktiv markiertem Lipid beruht auf einem Lipidaustausch zwischen Vesikel- und Zellmembran. Es wird darüber hinaus auch gezeigt, daß sehr kleine Mengen einer biologisch hochwirksamen Substanz (maximal 5%) 2',3'c-AMP in Maus-3T3- und Maus-L929-Zellen eingeschleust werden konnten, wobei die Wachstumshemmung dieser Zellen beobachtet wurde. Mit roten Blutzellen gelang die Einschleusung bisher nicht, da diese Zellen naturgemäss einer Fusion besonders hohen Widerstand entgegensetzen. Versuche, Stoffe in rote Blutzellen mit Hilfe von Vesikeln einzuschleusen, waren daher bis heute erfolglos. Dies ist auch der Grund dafür, daß die bisherigen Untersuchungsergebnisse ausschließlich die Wechselwirkung von Lipidvesikeln mit "Ghosts" betreffen, wie auch in C.A. Vol. 75 (1971). Es existieren bisher keine Ergebnisse, die an intakten roten Blutzellen eine "Rechtsverschiebung" der $O_2$-Bindungskurve über den Wert des DPG—Hb's ($P_{O_{2(1/2)}}$ = 23,7 mm Hg) beweisen. Gemäss der Erfindung wird eine "Rechtsverschiebung" bis zu einem $P_{O_{2(1/2)}}$ = 96,4 mm Hg erzielt. Dies ist nur möglich, wenn der stark wirksame Effektor IHP in Konzentrationen in den roten Blutzellen angereichert werden kann, die eine 1:1-Komplexbildung mit Hb ermöglicht. Niemand ist dies bisher gelungen.

Der Stand der Technik läßt sich folgendermaßen charakterisieren:

a) Lipidvesikel wurden bisher nur in "Ghosts" und nicht in intakte Erythrozyten inkorporiert. Eine Fusion der Lipid-Vesikel mit der Membran intakter Erythrozyten (eine Voraussetzung, die durch das vorliegende neue Verfahren gegeben ist) ist bisher nicht erfolgreich durchgeführt worden.

b) Die Wirkung von IHP auf Hämoglobin ist bekannt. Nicht bekannt war die Wirkung von IHP auf die intakte rote Blutzelle insgesamt und auf das intrazelluläre Hämoglobin.

c) Die Verwendung bisheriger Erkenntnisse und die Durchführung einfacher Vorversuche war nicht ausreichend, um die IHP-Einschleusung in rote Blutzellen und die Herabsetzung der $O_2$-Affinität der roten Blutzellen zu erreichen. Wäre dies so, dann hätte insbesondere für die Human-Medizin dieses außerordentlich wichtige Problem bereits früher gelöst werden können, zumal viele Forschungsinstitutionen bemüht waren, sowohl Lipid-Vesikel für den Transport nutzbar zu machen, als auch die $O_2$-Affinität des Hämoglobins in Erythrozyten zu modifizieren.

Das Erfinderische gegenüber dem Stand der Technik lässt sich wie folgt zusammenfassen:

a) Durch Fusion der Lipid-Vesikel, die IHP enthalten, mit der roten Blutzelle wird das stark negativ geladene Ion in die Zelle injiziert, um dann auf der Innenseite der Erythrozyten-Membran durch Wechselwirkung eine Umordnung der Erythrozyten-Membran herbeizuführen, die den schnellen Einstrom von extrazellulärem IHP ermöglicht. Diese Umordnung der Membran erlaubt nur den Durchtritt von relativ kleinen Molekülen, *nicht jedoch den Austritt vom Hämoglobin aus der Zelle.*

b) IHP wirkt in der Zelle als ein Effektor, der die Hämoglobinstruktur verändert und die $O_2$-Affinität des Hämoglobins herabsetzt. Seine Einzigartigkeit verdankt es nicht nur seiner hohen Affinität zum DPG-Bindungsort im Hämoglobin, sondern vor allem der Tatsache, daß es nicht im Erythrozyten abgebaut werden kann und aus der Zelle hinausgelangt.

c) Niemand hat bisher IHP-enthaltende Lipid-Vesikel für die unidirektionale Einschleusung von Stoffen verwendet.

Es wurde nun überraschenderweise gefunden, daß es mit Hilfe von geladenen fluiden Lipidvesikeln, die mit der Erythrozytenmembran zu fusionieren vermögen, möglich ist, allosterische Effektoren wie Inosithexaphosphat (IHP) in Erythrozyten zu transportieren, wo es aufgrund seiner viel höheren Bindungskonstante das 2,3-Bisphosphoglycerat an seiner Bindungsstelle im Hämoglobin verdrängt und irreversibel inkorporiert wird. Unter diesen Bedingungen geht das Hb in den Erythrozyten in eine allosterische Konformation mit einer bedeutend niedrigeren Affinität zu Sauerstoff über.

Die Verminderung der $O_2$-Affinität, d.h. die Erhöhung des $O_2$-Halbsättigungsdrucks von Hämoglobin in Erythrozyten, steigert die Fähigkeit der Erythrozyten, das gebundene $O_2$ selbst gegen höhere $O_2$-Partialdrücke zu dissoziieren und verbessert somit die Sauerstoffzufuhr zu den Geweben.

Erythrozyten, in die IHP inkorporiert worden ist und die daher eine verbesserte Sauerstoffzufuhr zu den Geweben bewirken, können in den folgenden Fällen Anwendung finden:

1) Bei niedrigem Sauerstoffpartialdurck in der Atemluft: Bergsteiger in großen Höhen, Astronauten in $O_2$-armer Atmosphäre.

2) Bei verringerter $O_2$-Austauschfläche: Verminderung der Zahl der Lungenalveolen bei Lungenemphysem.

3) Bei erhöhtem Widerstand gegen $O_2$-Diffusion in der Lunge: Pneumonie, Asthma.

4) Bei verringertem $O_2$-Transportvermögen: Erythropeny, anämische Zustände aller Art, arteriovenöser Shunt.

5) Bei Störungen der Blutzirkulation: Arteriosklerose, thromboembolische Prozesse, Organinfarkt, ischämische Zustände.

6) Bei hoher Affinität von Hämoglobin zu Sauerstoff: Hämoglobin-Mutationen, chemische Modifikation von N-endständigen Aminosäuren in den Hämoglobinketten, z.B. Diabetes mellitus, Enzymdefekte in Erythrozyten.

7) Zur Beschleunigung von Entgiftungsprozessen durch verbesserte Sauerstoffversorgung.

8) Zur Verminderung der Affinität von konserviertem Blut zu Sauerstoff: Transfusion, Schockzustände.

9) Zur Verbesserung der Radiotherapie von Krebs.

Als allosterische Effektoren für den Transport in die Erythrozyten kommen überwiegend solche infrage, die eine höhere Affinität zum Hämoglobin als die physiologischen Effektoren 2,3-Bisphosphoglycerat und Adenosintriphosphat aufweisen.

Insbesondere wird als allosterischer Effektor Inosithexaphosphat bevorzugt.

Als allosterischer Effektor kommt auch Inositpentaphosphat infrage.

Als Lipidvesikel werden Gemische von Phosphatidylcholin/Phosphatidylserin/Cholesterin im Molverhältnis von 10 bis 5:4 bis 1:10 bis 3 eingesetzt. Ganz besonders bevorzugt wird ein Molverhälnis von 8:2:7. Aber auch ein Molverhältnis von 9:1:8 sowie von 8:4:7 ist günstig.

Als fluide Trägerflüssigkeiten kommen die üblichen Trägerflüssigkeiten, insbesondere gepufferte physiologische Trägerflüssigkeiten infrage, die dem Fachmann ohne weiteres bekannt sind bzw. zur Verfügung stehen, wie isotonische bis-Tris-Puffer.

Die Lipidvesikel müssen in der Lage sein, mit der Membran der Erythrozyten zu fusionieren. Durch die Inkorporierung der allosterischen Effektoren in intakte Erythrozyten ist es möglich geworden, daß das Hämoglobin in den Erythrozyten in eine allosterische Konformation übergeht, die wesentlich leichter Sauerstoff abgibt.

Die Erfindung macht es möglich, modifizierte Erythrozyten zur Verfügung zu stellen, die eine verbesserte Sauerstoffausnutzung des Blutes gewährleisten. Erhalten werden diese modifizierten Erythrozyten, indem man in sie mit Hilfe von Lipidvesikeln allosterische Effektoren irreversibel inkorporiert, wobei die Lipidvesikel mit den Erythrozytenzellmembranen fusionieren und die allosterischen Effektoren an das Hämoglobin in den Erythrozyten gebunden wird.

Wenn beispielsweise IHP als allosterischer Effektor verwendet wird, werden zunächst Lipidvesikel mit dem IHP beladen und dann mit den Erythrozyten fusioniert, wobei das IHP an das Hämoglobin gebunden wird, wodurch sich die allosterische Konformation des Hämoglobins und damit dessen Affinität zu Sauerstoff ändert.

Das Inkorporieren der Kombination von allosterischen Effektoren und Lipidvesikeln in die Erythrozyten erfolgt extrakorporal. Dementsprechend umfasst die Erfindung auch Arzneimittel bzw. die Anwendung der modifizierten Erythrozyten bei der Bekämpfung der verschiedenen Krankheiten, wie sie vorstehend erwähnt worden sind.

Bei der Anwendung und Verwendung werden von entnommenem Blut abgetrennte Erythrozyten durch Inkorporieren der Kombination von Lipidvesikeln mit allosterischen Effektoren modifiziert und die modifizierten Erythrozyten dem Blutplasma wieder zugeführt.

Daher ist es ohne weiteres möglich, Blutkonserven oder Blut mit modifizierten Erythrozyten zu erhalten, die überall dort eingesetzt werden können, wo es nötig ist, eine verstärkte Sauerstoffabgabe in der Endstrombahn zu erreichen bzw. die Sauerstoffausnutzung des Gewebes zu erhöhen.

Die modifizierten Erythrozyten können auch in einer physiologisch geeigneten Trägerflüssigkeit intravenös dem Blutkreislauf eines Warmblüters zugeführt werden.

Eine spezielle Ausführungsform der Herstellung von modifizierten Erythrozyten wird nachstehend geschildert.

Modifizierte Erythrozyten mit verbesserter Sauerstoffabgabe werden dadurch erhalten, daß man

a) Inosithexaphosphat in einer isotonischen Pufferlösung löst, bis die Lösung gesättigt ist,

b) in dieser Lösung ein Lipidgemisch suspendiert, das Phosphatidylcholin, Phosphatidylserin und Cholesterin im Molverhältnis von 10 bis 5:4 bis 1:10 bis 3 enthält,

c) die erhaltene Suspension der Desintegration mit Ultraschall oder einem Einspritzverfahren unterwirft,

d) das Gemisch zentrifugiert und hierdurch die überstehende Suspension abtrennt, die die mit Inosithexaphosphat angereicherten kleinen Lipidvesikel sowie freies Inosithexaphosphat enthält,

e) worauf man menschliche Erythrozyten, die vorher vom Blutplasma durch Zentrifugieren abgetrennt worden sind in der überstehenden Suspension suspendiert, die die mit Inosithexaphosphat angereicherten kleinen Lipidvesikel sowie freies Inosithexaphosphat enthält,

f) die erhaltene Suspension inkubiert und hierdurch die Vesikel mit den Erythrozyten fusioniert, und

g) die nunmehr modifizierten intakten Erythrozyten mit isotonischer Kochsalzlösung oder isotonischem Puffer wäscht und quantitativ von außen vorhandenem freiem Inosithexaphosphat befreit und im Blutplasma bzw. Blutplasma-Ersatzlösungen suspendiert.

Die Erfindung wird nachstehend unter Bezugnahme auf die Abbildungen erläutert, wobei mit RBC rote Blutzellen bezeichnet werden.

Fig. 1 ist eine graphische Darstellung, die die Steigerung der $O_2$-Affinität von Hämoglobin in den Erythrozyten mit der Aufbewahrungszeit bei 4°C veranschaulicht. Der $O_2$-Halbsättigungsdruck ($P_{O_2(1/2)}$ wurde bei 25°C in Abwesenheit von $CO_2$ gemessen. In ACD konserviertes RBC —⊠—⊠—⊠—; RBC, das in isotonischem 0,10-molarem Bis-tris-Puffer von pH 7,4, der 0,154 mol/l NaCl enthält, aufbewahrt worden ist.

Fig. 2 veranschaulicht die "Linksverschiebung" der $O_2$-Bindungskurven von Hämoglobin in Erythrozyten in Abhängigkeit von der Aufbewahrungszeit bei 4°C. RBC wurde in isotonischem 0,10-

molarem Bis-tris-Puffer von pH 7,4, der 0,154 mol/l NacL enthält, bei 4°C aufbewahrt. Die Bindungsisothermen wurden bei 25°C in Abwesenheit von $CO_2$ gemessen. ————————: RBC, das vollständig von Polyphosphaten befreit ist; — — — — — — — — — — —: RBC bei der Halbwertszeit der Polyphosphatverarmung ($\tau_{1/2}$ = 9 d); —.—.—.—.—: frisches RBC. Der Pfeil deutet die Entladung des Hämoglobins unter dem $O_2$-Partialdruck von 30 Torr an.

Fig. 3 veranschaulicht die pH-Abhängigkeit der $O_2$-Bindungskurven bei 25°C in Abwesenheit von $CO_2$. RBC wurde 17 Tage bei 4°C in isotonischem 0,10-molarem Bis-tris-Puffer von pH 7,4, der 0,154 mol/l NaCl enthält, aufbewahrt: — — — — — — — — —, pH 7,72; ————————: pH 7,42; —.—.—.—.—: pH 7,08, Der Pfeil deutet die verminderte Sättigung des Hämoglobins unter einem $O_2$-Partialdruck von 30 Torr an.

Fig. 4 veranschaulicht den Bohr-Effekt von Hämoglobin in Erythrozyten bei 25°C in Abwesenheit von $CO_2$. RBC wurde 17 Tage bei 4°C in isotonischem 0,10-molarem Bis-tris-Puffer von pH 7,4, der 0,154 mol/l NaCl enthält, aufbewahrt.

Fig. 5 veranschaulicht die irreversible Inkorporierung von IHP in rote Blutzellen. Der $O_2$-Halbsättigungsdruck der Erythrozyten wurde bei 25°C in Abwesenheit von $CO_2$ vor und nach der mit V2 vermittelten Inkorporierung von IHP bei pH 7,6 gemessen. Das RBC wurde bei 4°C in isotonischem 0,10-molarem bis-Tris-Puffer von pH 7,4, der 0,154 mol/l NaCl enthält, aufbewahrt. —O—O—O—: Polyphosphat-Verarmungskurve von RBC; □⟵⟶□: Beladung mit IHP bei pH 7,6 und Aufbewahrung des mit IHP beladenen RBC bei 4°C; Δ⟵⟶Δ: Beladung mit IHP bei pH 7,6 und Aufbewahrung des mit IHP beladenen RBC bei 37°C. Die Inkorporierung von IHP wurde unter Standardbedingungen vorgenommen.

Fig. 6 veranschaulicht den Bohreffekt der mit IHP beladenen Erythrozyten bei 25°C in Abwesenheit von $CO_2$. —O—O—O—: Durch V2 vermittelte Beladung mit IHP bei pH 7,6; —□—□—□—: Durch V2 vermittelte Beladung mit IHP bei pH 7,8. Das RBC wurde bei 4°C in isotonischem 0,10-molarem bis-Tris-Puffer von pH 7,4, der 0,154 mol/l NaCl enthält, aufbewahrt. Die Beladung mit IHP erfolgte unter Standardbedingungen.

Fig. 7 veranschaulicht den Einfluß der Zusammensetzung der Vesikel auf die "Rechtsverschiebung der $O_2$-Bindungsisothermen, gemessen für pH 7,6 bei 25°C in Abwesenheit von $CO_2$. Die Beladung mit IHP erfolgte bei pH 7,6, unter Standardbedingungen. Die Zellen wurden in isotonischem Puffer von pH 7,6 suspendiert. —.—.—.—.—: RBC wurde 19 Tage in isotonischem 0,10-molarem bis-Tris-Buffer von pH 7,4, der 0,154 mol/l NaCl enthielt, auf bewahrt; — — — — — — — — — — —: RBC nach der durch V1 vermittelten Beladung mit IHP; —..—..—: frisches RBC; ————————: RBC nach der mit V2 vermittelten Beladung mit IHP. Der Pfeil deutet die verminderte Sättigung des Hämoglobins unter dem $O_2$-Partialdruck von 30 Torr an.

Fig. 8 veranschaulicht die Stabilität von mit IHP beladenen Vesikeln bei 37°C. —O—O—O—: $V_2$-Vesikel; —□—□—□—: V3-Vesikel. Durch Vesikel vermittelte Aufnahme von IHP durch RBC erfolgte bei pH 7,4 unter Standardbedingungen. $P_{O_{2(1/2)}}$ der mit IHP beladenen Erythrozyten wurde bei 25°C in Abwesenheit von $CO_2$ gemessen.

Fig. 9 veranschaulicht die Kinetik der Inkorporierung von $^{14}C$-Cholesterin aus Vesikeln in intakte Erythrozyten. Die Inkubation erfolgte bei 37°C und pH 7,4 in isotonischem 0,1-molarem bis-Tris-Puffer, der 0,154 Mol NaCl enthielt.

Fig. 10 veranschaulicht die Kinetik der Inkorporierung von $^{14}C$-Cholesterin aus V2-Vesikeln in intakte Erythrozyten. Die Radioaktivität wurde in mit Folch extrahierten Lipiden der Erythrozyten gemessen. Die Inkubation erfolgte unter den für Fig. 9 genannten Bedingungen.

Fig. 11 veranschaulicht die Kinetik der Aufnahme von $^{14}C$-Cholesterin durch Helazellen aus Lipidvesikeln. Die Inkubation erfolgte bei 37°C in phosphatgepufferter Kochsalzlösung bei pH 7,4.

Fig. 12 veranschaulicht die pH-Abhängigkeit der $^{14}C$-Cholesterinaufnahme durch Hela-Zellen aus Vesikeln. Die Bedingungen waren die gleichen wie in Fig. 11.

Fig. 13 veranschaulicht den Einfluß des pH-Werts des Inkubationsmediums auf den $O_2$-Halbsättigungsdruck von mit IHP beladenen Erythrozyten. $P_{O_{2(1/2)}}$ wurde bei 25°C in Abwesenheit von $CO_2$ gemessen.

Fig. 14 veranschaulicht die Kinetik der durch V2 vermittelten Beladung von Erythrozyten mit IHP bei pH 7,38 in Gegenwart von IHP im äußeren Medium. Die Inkubation von 57 Tage altem RBC erfolgte unter Standardbedingungen. $P_{O_{2(1/2)}}$ wurde bei 25°C in Abwesenheit von $CO_2$ gemessen.

Fig. 15 veranschaulicht den Einfluß des pH-Werts des Inkubationsmediums auf die Halbwertzeit der durch V2 vermittelten Inkorporierung von IHP. Das RBC wurde unter Standardbedingungen mit IHP im äußeren Medium inkubiert. Der $P_{O_{2(1/2)}}$ wurde bei 25°C in Abwesenheit von $CO_2$ gemessen.

Fig. 16 veranschaulicht die Kinetik der durch V2 vermittelten Beladung von Erythrozyten mit IHP bei pH 7,4 in Abwesenheit von IHP im äußeren Medium. Die Inkubation erfolgte unter Standardbedingungen. $P_{O_{2(1/2)}}$ wurde bei 25°C in Abwesenheit von $CO_2$ gemessen.

Fig. 17 zeigt die $O_2$-Bindungskurven vor und nach der durch V2 vermittelten IHP-Beladung von Erythrozyten bei pH 7,4. Die Inkubation erfolgte unter Standardbedingungen bei pH 7,6. Die Sauerstoffabgabe durch die Erythrozyten gegen den für das Gehirn kritischen $O_2$-Partialdruck von 30 Torr ist

durch Pfeile angedeutet: bei 25°C: ————, Erythrozyten 41 Tage alt ($O_2$-Abgabe 5%); – – – – – – –, mit IHP beladene Erythrozyten ($O_2$-Abgabe 47%); Bei 37°C: —.—.—.—, 41 Tage alte Erythrozyten ($O_2$-Abgabe 21%); —...—...: mit IHP beladene Erythrozyten ($O_2$-Abgabe 21%); —...—...: mit IHP beladene Erythrozyten ($O_2$-Abgabe 80%).

Der Grad der Sauerstoffabgabe durch das in den Erythrozyten enthaltene Hämoglobin in den Kapillaren hängt nicht nur vom $O_2$-Partialdruck des venösen Bluts, sondern vor allem von der Affinität des Hämoglobins in den roten Zellen zu Sauerstoff ab. Innerhalb der Erythrozyten enthaltene allosterische Effektoren, die die Affinität zu Sauerstoff regeln, sind die Bohr Protonen, $CO_2$ und organische Phosphatverbindungen, insbesondere DPG (2,3-Bisphosphoglycerat, natürlicher allosterischer Effektor) und ATP (Adenosintriphosphat).

Die Verarmung aufbewahrter roter Zellen an DPG und ATP führt zu einem allmählichen Anstieg der Affinität zu Sauerstoff (S. Balcerzak et al: Adv.Exp.Med.Biol.28 (1972) 433—447). Sie wird in Fig. 1 graphisch durch Darstellung des $O_2$-Partialdrucks bei Halbsättigung (gemessen bei 25°C) in Abhängigkeit von der Aufbewahrungszeit veranschaulicht (Erythrozyten bei 4°C aufbewahrt). Die $O_2$-Bindungsisothermen werden in Abwesenheit von $CO_2$ bei konstantem pH-Wert (pH 7,4) gemessen, um die Einflüsse dieser allosterischen Effektoren auf den Halbsättigungsdruck auszuschließen. Der Endpunkt der zeitabhängigen Verarmung an Polyphosphat ist durch $P_{O_{2(1/2)}} = 4,2$ Torr, definiert, d.h. den Halbsättigungsdruck von vollständig polyphosphatfreiem (stripped) Hämoglobin. Der Startpunkt, d.h. $P_{O_{2(1/2)}}$ von frischen Erythrozyten, hängt von der Zusammensetzung des Suspensionsmediums ab. Aus diesen Polyphosphatverarmungskurven kann ein neuer functioneller Parameter von aufbewahreten Erythrozyten, die sog. Halbwertzeit der Polyphosphatverarmung innerhalb der Erythrozyten, bestimmt werden. Sie beträgt 9 Tage in isotonischem 0,1-molarem bis-Tris-Puffer von pH 7,4 und 12 Tage in ACD (Zitronensäure-Natriumcitrat-Dextrose: Aufbewahrungslösung) als Stabilisatorlösung.

Die Verarmung an Polyphosphaten in Erythrozyten verursacht eine "Linksverschiebung" der $O_2$-Bindungsisothermen und damit eine Verringerung des Sauerstoffabgabevermögens.

Die "Linksverschiebung" der $O_2$-Bindungskurve und die Abnahme des Sauerstoffabgabevermögens, gemessen bei 25°C, von bei 4°C aufbewahrten Erythrozyten sind in Fig. 2 dargestellt. Unter einem $O_2$-Partialdruck von 30 Torr werden frische Erythrozyten zu 11% entsättigt, während Erythrozyten, die an Polyphosphaten halb verarmt sind, nur zu 1% entsättigt werden.

Die "Linksverschiebung" der $O_2$-Bindungskurven von an Polyphosphat verarmten Erythrozyten verursacht eine Beeinträchtigung der Sauerstoffabgabe in den Geweben. Eine massive Transfusion von gelagertem Blut (mit DPG-Mangel) führt somit zu einer Senkung des Muskel-pH-Werts und einem Anstieg des Lactatspiegels im Plasma, begleitet von einem als "Transfusionssyndrom" bekannten Abfall des Blutdrucks (S. V. Kevy und Mitarbeiter, Adv. Exp. Med. Biol. 28 (1972) 511—516). Diese Senkung des pH-Werts gleicht die in Fig. 3 dargestellte "Linksverschiebung" nur teilweise aus. Bei einem $O_2$-Partialdruck von 30 Torr steigert eine Verschiebung des pH-Werts von 7.42 nach 7,08 die Sauerstoffabgabe der roten Zellen (die 17 Tage bei 4°C in 0,1-molarem isotonischem bis-Tris-Puffer von pH 7,4 gelagert worden sind) von 1% auf 5%; aber eine Abgabe bis 11%, die bei frischen roten Zellen bei pH 7,46 beobachtet wird, kann durch den Bohr-Effekt allein nicht bewirkt werden (siehe (Fig. 2). Der Einfluß des pH-Werts auf die $O_2$-Affinität (Bohr-Effekt gelagerter roter Zellen, die in isotonischem 0,1-molarem bis-Tris-Puffer bzw. Tris-Puffer suspendiert sind, ist in Fig. 4 für den Bereich von pH 7,0 bis 7,8 dargestellt. Die $O_2$-Bindungskurven wurden bei 25°C in Abwesenheit von $CO_2$ mit Erythrozyten gemessen, die 17 Tage bei 4°C gehalten worden waren. Diese gealterten Erythrozyten haben mehr als die Hälfte ihres Polyphosphat-Effekts auf die Affinität von Hämoglobin zu Sauerstoff verloren (siehe Fig. 1). Der Bohr-Effekt $-\Delta\ P_{O_{2(1/2)}}\ \Delta pH$ entspricht 0,53 Protonen pro Mol $O_2$. Die Zahl der unter Sauerstoffbindung abgegebenen Bohr-Protonen ist wenigstens bis zu einem RBC-Alter von 34 Tagen konstant, wenn be eits 75% des Polyphosphat-Effekts verlorengegangen ist. Daher hat die Lagerung, d.h. die Verarmung an Polyphosphaten, keinen Einfluß auf den Bohr-Effekt der Erythrozyten.

Methoden

*Entnahme und Lagerung von menschlichem Blut*

Einer jungen gesunden freiwilligen Versuchsperson wurden 100 ml Blut entnommen, das in einem 250 ml-Biokolben (Biotest-Serum-Institut, Frankfurt am Main), der 50 ml ACD als Stabilisator enthielt, aufgefangen wurde. Diese Probe wurde bei 4°C aufbewahrt.

*Gewinnung und Aufbewahrung von menschlichen Erythrozyten*

300 ml Blut von einem jungen Probanden wurden in einem Kunststoffbeutel aufgefangen, der zur Verhinderung der Blutgerinnung Heparin oder Natriumcitrat enthielt. Die Blutprobe wurde im Eisbad gekühlt. Die weitere Behandlung wurde bei 4°C vorgenommen. Die Erythrozyten wurden vom Plasma durch Zentrifugieren für 20 Minuten bei 23500 xg abgetrennt (Sorvall, Typ RC—2B; Rotor SS 34; 12000 UpM). Die gepackten roten Zellen wurden in isotonischem bis-Tris-Puffer von pH 7,4 (0,10 M bis-Tris, 0,154 M NaCl) suspendiert und zentrifugiert. Diese Wäsche wurde dreimal wiederholt.

Abschließend wurden die sedimentierten Erythrozyten in 300 ml isotonischem bis-Tris-Puffer von pH 7,4 suspendiert und bei 4°C gelagert.

*pH-Messung und Einstellung der Erythrozyten-Suspension*

Die in isotonischem 0,1-molarem Puffer von pH 7,4 bzw. in ACD gehaltenen roten Zellen wurden bei Raumtemperatur 2 Min. bei 8000 xg zentrifugiert (Eppendorf-Zentrifuge, Typ 3200, 12000 UpM). Die Erythrozyten-Suspension wurde durch wiederholtes Auswechseln des Puffermediums auf den gewünschten pH-Wert eingestellt. Die Zellen wurden im gewünschten Puffer suspendiert und erneut zentrifugiert. Diese maßnahme muß wiederholt werden, bis ein konstanter pH-Wert erreicht ist.

Die pH-Messung erfolgt bei 25°C mit einer Glaselektrode (Ingold, Frankfurt a.M., Typ 406—M3, a = 35 mm). Die Genauigkeit der pH-Messung beträgt ± 0,02 Einheiten.

*Herstellung der mit IHP beladenen Lipid-Vesikel*

IHP wurde zwischen Raumtemperatur und 50°C in isotonischem bis-Tris-Puffer (0,10 M bis-Tris, 0,154 M NaCl) von pH 7,4 bis zur Sättigung (0,19 M) gelöst. Ein aus Phosphatidylcholin (PC), Phosphatidylserin (PS) und Cholesterin (Ch) im Molverhältnis von 8:2:7 (siehe Tabelle 1) bestehendes Lipidgemisch wurde in dieser Lösung suspendiert und 45 Minuten unter Stickstoff bei etwa 50°C beschallt. Der Temperaturbereich für die Herstellung der Vesikel ist nur durch den Gefrierpunkt des Puffers und die thermische Stabilität des Polyphosphats begrenzt. Die Beschallung wurde mit einem Ultraschall-Desintegrator (Schöller, Typ 125, Frankfurt a.Main) mit einer Titan-Tauchsonde (10 kHz) vorgenommen. Die Beschallung kann wirksam bei vorzugsweise über 100 $W/cm^2$ liegenden Energien erfolgen. Nach der Beschallung wurde die Vesikelsuspension 1 Std. bei 100 000xg bei 25°C in einer Ultrazentrifuge zentifugiert (Beckmann, Typ L5—65, Rotor 60). Der Überstand enthielt die kleinen Lipidvesikeln mit einem Durchmesser von ⩽500 Å. Bei der Bildung der Vesikel schließen sie die Lösung ein, in der die Lipide suspendiert werden.

TABELLE 1

Zusammensetzung der Lipid-Vesikel*

| Vesikel | Molverhältnisse PC : PS : CH |
|---|---|
| V1 | 9 : 1 : 8 |
| V2 | 8 : 2 : 7 |
| V3 | 8 : 4 : 7 |
| V4 | 8 : 0 : 7 |

* Brauchbar sind Molverhältnisse im Bereich (V1—V3) von PC:PS:CH = 10—5:4—1:10—3.

Phosphatidylserin wurde aus Rinderhirn (Koch-Light, England) und Phosphatidylcholin aus Eigelb (Lipid Specialties, Boston, USA) hergestellt. Cholesterin und das Natriumsalz von Inosithexaphosphat wurden von Merck (Darmstadt) bzw. Sigma (München) bezogen. Alle Lipide wurde durch Säulenchromatographie gereinigt. Ihre Reinheit wurde durch Dünnschichtchromatographie nachgeprüft.

1n mol/l Lipid ergibt 2 × $10^{11}$ Lipid-Vesikel, die mit doppelschichtigen Lipid-Membranen versehen sind (kleine Vesikel mit einem Durchmesser von 500 Å und weniger). 2 × $10^{11}$ Lipid-Vesikel wurden mit $10^6$ Erythrozyten inkubiert, jedoch wurden auch andere Verhältnisse, die nachstehend genannt sind, angewendet.

*Inkorporierung von Inosithexaphosphat in menschliche Erythrozyten*

Für In-vitro-Versuche wurden 200 μl Erythrozytensuspension bei Raumtemperatur 2 Minuten bei 8000 xg (Eppendorf-Zentrifuge, Typ 3200, 12000 UpM) zentrifugiert. Falls erforderlich, wurden die sedimentierten Zellen in der oben beschriebenen Weise gewaschen und auf den gewünschten pH-Wert eingestellt und dann erneut in 200 μl isotonischem 0,1-molaren Puffer vom gewünschten pH-Wert suspendiert. Der Suspension wurde ein gleiches Volumen der Lipid-Vesikelsuspension vom gewünschten pH-Wert zugesetzt. Die Erythrozyten wurden 1 Stunde bei 37°C inkubiert. Die Erythrozyten wurden dann mehrmals mit 0,1-molarem isotonischem Puffer gewaschen, bis ein

konstanter pH-Wert erreicht war. Fällungsversuche mit $Ca^{+2}$ wurden mit dem Überstand durchgeführt, bis kein freies IHP mehr nachgewiesen werden konnte. Beliebige Puffersysteme, die im pH-Bereich von 7 bis 8 wirksam sind und die einwandfreien strukturellen, morphologischen und funktionellen Eigenschaften der Erythrozyten nicht beeinträchtigen, können verwendet werden.

*Messung der $O_2$-Bindungskurven*

Die $O_2$-Bindungskurven wurden bei 25°C mit Hilfe der schnellen Diffusionsmethode (H. Sick und K. Gersonde, Analyt. Biochem. 32 (1969) 362—376 und H. Sick und K. Gersonde, 47 (1972) 46—56) aufgenommen.

Ergebnisse

*Bohr-Effekt von gelagerten menschlichen roten Zellen nach Fusion mit Vesikeln*

Der $O_2$-Halbsättigungsdruck, die Kooperativität (n = 2,8) und der Bohr' Effekt von menschlichen Erythrozyten, die 40 Tage in isotonischem Puffermedium von pH 7,4 bie 4°C gehalten worden sind und teilweise an Polyphospaten verarmt sind, werden nach Inkubation in isotonischem Puffer von pH 7,6 mit den Vesikeln V2 bei 37°C für 1 Stunde nicht wesentlich verändert. Die Inkubation von Erythrozyten mit Vesikeln mit unterschiedlicher Lipidzusammensetzung (V1 und V3) hat ebenfalls keinen Einfluß auf die $O_2$-Bindungsparameter des Hämoglobins innerhalb der Erythrozyten.

*Bohr-Effekt von aufbewahreten menschlichen roten Blutzellen nach der durch Vesikel (V2) vermittelten Inkorporierung von Inosithexaphosphat*

IHP, der stärkste bisher bekannte allosterische Effektor von Hämoglobin zu Sauerstoff, erkennbar an einer "Rechtsverschiebung" der $O_2$-Bindungskurve. Menschliche Erythrozyten, die nicht in der Lage sind, IHP zu synthetisieren, können mit diesem Polyphosphat während der Fusion mit Lipid-Vesikeln, die den Effektor enthalten, beladen werden.

Menschliche Erythrozyten, die 25 Tage bei 4°C in isotonischem Puffermedium von pH 7,4 gehalten worden waren ($P_{O_2(1/2)}$ = 6,0 Torr), wurden auf pH 7,6 eingestellt ($P_{O_2(1/2)}$ = 4,5 Torr) und dann 1 Stunde bei 37°C in isotonischem 0,1-molarem Tris-Puffer von pH 7,6, der 0,19 mol/ IHP enthielt, mit V2-Vesikeln, die mit IHP beladen waren, inkubiert. Nach Einbau von IHP in die Erythrozyten stieg der $O_2$-Halbsättigungsdruck drastisch ($P_{O_2(1/2)}$ = 14,3 Torr) um einen Faktor von 3,2 an und überschritt den Wert für frische Erythrozyten ($P_{O_2(1/2)}$ = 10,55 Torr) um einen Faktor von 1,4.

Dann wurden diese mit IHP beladenen Erythrozyten 6 Tage bei 4°C in isotonischem Puffermedium von pH 7,6 gehalten. Der Halbsättigungsdruck blieb konstant. Nach weiteren 4 Tagen bei 4°C wurde der pH-Wert der mit IHP beladenen Erythrozyten auf 7,28 geändert, wobei der $P_{O_2(1/2)}$ wieder auf 32,1 Torr stieg. Bei weiteren Versuchen wurden gewaschene Erythrozyten, die 36 Tage bei 4°C gehalten worden waren (stärker an DPG verarmt), auf pH 7,6 eingestellt und in der oben beschriebenen Weise mit IHP beladen. Auch hier stieg der $P_{O_2(1/2)}$ auf 14,0 Torr. Die mit IHP beladenen Zellen wurden 2 Tage bei 37°C gehalten. Auch wurde keine Veränderung der Affinität zu Sauerstoff nach Lagerung dieser mit IHP beladenen Zellen für 2 Tage bei 37°C festgestellt. Im Gegensatz zu normalen Erythrozyten, die während der Lagerung bei 4°C eine Abnahme der physiologischen Polyphosphate un die Hälfte zeigen, scheinen mit IHP beladene Erythrozyten das IHP wenigstens während einer Zeit von 9 Tagen nicht zu hydrolysieren, erkennbar an einem konstanten $P_{O_2(1/2)}$-Wert (siehe Fig. 5). Mit den in dieser Weise behandelten Erythrozyten können somit wesentlich längere Konservierungszeiten erreicht werden.

Darüber hinaus zeigen die mit IHP beladenen roten Blutzellen einen stärkeren Bohr-Effekt als die unbehandelten Zellen (Siehe Fig. 6).

In Abwesenheit von $CO_2$ beträgt der Bohr-Effekt von Erythrozyten, die mit IHP-beladenen V2-Vesikeln in Gegenwart von freiem IHP bei pH 7,6 inkubiert und durch Waschen mit dem jeweiligen isotonischen 0,1-molaren Puffer auf den gewünschten pH-Wert eingestellt worden sind, $-\Delta P_{O_2(1/2)}$ $\Delta$ pH = 1,20 Protonen pro Mol $O_2$. Die Bohr-Protonenangabe von IHP-beladenen Erythrozyten ist dreimal größer als in normalen frischen roten Blutzellen. Daher machen IHP-beladene Zellen die Sauerstoffabgabe in den Geweben und die Sauerstoffaufnahme in der Lunge wirksamer. Die Inkorporierung von IHP mit IHP-beladene V2-Vesikeln in Gegenwart von freiem IHP ist bei pH 7,8 viel wirksamer. Nachdem der pH-Wert der IHP-beladenen Zellen auf 7,4 geändert worden ist, kann der theoretisch erwartete Anstieg auf $P_{O_2(1/2)}$ = 35 Torr beobachtet werden (siehe Fig. 6).

Andererseits scheint der Bohr-Effekt nach dem Einbau bei pH 7,8 kleiner zu werden ($-\Delta P_{O_2(1/2)}/\Delta$ pH = 0,9 Protonen pro Mol $O_2$).

*Einfluß der Vesikelzusammensetzung auf die Aufnahme von Inosithexaphosphat durch menschliche Erythrozyten*

Die "Rechtsverschiebung" der $O_2$-Bindungskurve ist ein Maß der durch menschliche Erythrozyten aufgenommenen IHP-Menge. Der unter Standardbedingungen gemessene $O_2$-Halbsättigungsdruck ist somit ein Ausdruck des Wirkungsgrades des Einbaues von IHP. Dieser Wirkungsgrad der IHP-Aufnahme hängt weitgehend von der Lipidzusammensetzung der Vesikel ab.

In Fig. 7 ist die "Rechtsverschiebung" der $O_2$-Bindungskurve für die V1- und V2-Vesikel nach Inkubation bei pH 7,6 dargestellt. Die V2-Vesikel zeigen die stärkste "Rechtsverschiebung" bei einer Entladung von 14% bei 30 Torr. 19 Tage alte Erythrozyten zeigen keine Entladung bei diesem $O_2$-Druck, während frische rote Blutzellen sich bis 5% entladen. Die Inkorporierung von IHP mit Hilfe von V2-Vesikeln verbessert die Sauerstoffabgab der normalen roten Blutzellen bei 30 mm Hg um einen Faktor von etwa 3. V2- und V3-Vesikel bewirken eine gleiche IHP-Aufnahme durch Erythrozyten und daher gleiche "Rechtsverschiebungen" der $O_2$-Bindungskurven.

In Abwesenheit von IHP im äußeren Medium unterschieden sich V1-, V2- und V3-Vesikel, die IHP aufgenommen haben, hinsichtlich ihrer Halbwertzeit der Aufnhame nicht; $\tau_{1/2}$ beträgt 30 Minuten. Die V1- und V3-Vesikel zeigen nur weniger als die Hälfte des IHP-Effekts, gemessen für dialysierte, IHP-beladene V2-Vesikel.

Dies läßt eine verminderte Stabilität der V1- und V3-Vesikel erkennen. In Fig. 8 wird der Unterschied in der Stabilität zwischen V2- und V3-Vesikel veranschaulicht. Die Halbwertzeit der Stabilität beträgt bei den V2-Vesikeln etwa 3 Tage und bei den V3-Vesikeln etwa 1,5 Tage.

*Inkorporierung der Vesikel in die Blutzellen*

Die Aufnahme der Lipidvesikel in intakte Erythrozyten wurde mit den mit $^{14}C$-Cholesterin oder $^{14}C$-Phosphatidylcholin markierten Vesikeln V1, V2 und V3 verfolgt. Dies wurde mit der Aufnahme der gleichen Vesikel in kultivierte Hela-Zellen verglichen. Die Radioaktivität wurde sowohl in den intakten Erythrozyten (durch Löslichmachen und Bleichen mit einem Lumac-Reagens-Set) und in ihren Gesamtlipidextrakten (Folch) gemessen. Die Inkorporierung wurde während einer Zeit von 4 Stunden verfolgt. Die Ergebnisse sind in Fig. 9 und Fig. 10 dargestellt. Fig. 9 zeigt die Daten für die Vesikel V1, V2 und V3 mit intakten Erythrozyten. Das Inkubationsmedium enthielt 10 ml RBC (rote Blutzellen) und 10 ml IHP-beladene Vesikel in isotonischem 0,1-molarem bis-Tris-Puffer von pH 7,4. Aliquote Teile wurden nach 10, 20, 40, 60, 90, 120, 180 und 240 Minuten genommen und gezählt. Die Halbwertzeit der Inkorporierung beträgt 45 Minuten für die V2-Vesikel (die auch die höchste Radioaktivität im RBC zeigen) und 35 Minuten für die V1- und V3-Vesikel. Es ist zu betonen, daß die im RBC gefundene Radioaktivität nicht unbedingt die Aufnhame von Vesikeln bedeutet, da es wohlbenkannt ist, (B. Bloj und D. Zilversmit, Biochemistry 16 (1977) 3943—3948), daß Cholesterin zwischen Vesikeln und Erythrozyten ausgetauscht wird. In den RBC-Lipidextrakten (siehe Fig. 10) wurde ein $\tau_{1/2}$-Wert von 30 Minuten bei Verwendung von V2-Vesikeln gefunden. Wenn V2- und V3-Vesikel in Hela-Zellen inkorporiert werden, ergibt sich das gleiche Bild (siehe Fig. 11). Die Inkubation der Hela-Zellen mit den Vesikeln erfolgte unter den oben beschriebenen Bedingungen. Bei einer weiteren Versuchsreihe wurden Hela-Zellen, mit $^{14}C$-Cholesterin enthaltenden Vesikeln in isotonischen Puffern bei verschiedenen pH-Werten zwischen 7 und 8 inkubiert. Fig. 12 zeigt, daß die pH-Änderungen zwischen 7 und 8 nur wenig Einfluß auf die Inkorporierung des markierten Lipids in die Zellen hatten.

Die Halbwertzeit der Radioaktivitätsaufnahme durch die Erythrozyten war, wenn diese mit radioaktiv markierten Vesikeln inkubiert wurden, die gleiche wie die Halbwertzeit der IHP-Aufnahme durch Erythrozyten, die mit dialysierten, IHP-beladenen Vesikeln inkubiert wurden (Fig. 16). Dies ist ein zusätzlicher Beweis, daß von der nicht nur der Lipidaustausch zwischen Blutzellen und Vesikeln, sondern die Fusionierung von Vesikeln mit den Blutzellen gemessen wurde.

Dünnschichtchromatogramme des Lipidextrakts der Erythrozyten zeigten die Anreicherung der RBC-Membranlipide mit den Lipiden der Vesikel.

*Scheinbares pH-Optimum der durch V2 vermittelten IHP-aufnahme durch gelagerte menschliche rote Blutzellen*

Die Aufnahme von IHP durch gewaschene Erythrozyten hängt vom pH-Wert des Inkubationsmediums ab. Die graphische Darstellung von $P_{O_{2(1/2)}}$ von IHP-beladenen Erythrozyten in Abhängigkeit vom pH-Wert des Inkubationsmediums (siehe Fig. 13) zeigt ein deutliches pH-Optimum der Aufnahme von IHP im Bereich von pH 7,4 bis 7,5. Die Verminderung von $P_{O_{2(1/2)}}$ oberhalb von pH 7,5 entspricht der in Fig. 6 dargestellten Kurve des Bohr-Effekts und steht in Wechselbeziehung zur Verminderung der Affinität von IHP zu Hämoglobin. Unterhalb von pH 7,4 läßt der drastische Abfall von $P_{O_{2(1/2)}}$ eine begrenzte IHP-Aufnahme erkennen, so daß der theoretisch erwartete $P_{O_{2(1/2)}}$ für vollständig umgewandeltes Hämoglobin durch gebundenes IHP nicht beobachtet wird.

Die Anderung des pH-Werts des Puffermediums auf 7,4 nach Inkubation bei pH 7,8 erhöht den $P_{O_{2(1/2)}}$-Wert von Hämoglobin innerhalb der Erythrozyten auf Werte von 30 bis 40 Torr bei 25°C, wie in Fig. 6 veranschaulicht. Die Inkubation bei pH 7,8 und das Umpuffern auf verschiedene pH-Werte führen zu einem Anstieg von $P_{O_{2(1/2)}}$ über den gesamten pH-Bereich. Dieses Ergebnis läßt die Schlußfolgerung zu, daß der Einbau von IHP in Erythrozyten oberhalb von pH 7,4 wirksamer ist, obwohl er bei niedrigeren pH-Werten ebenfalls wirksam ist.

*Kinetik der IHP-Aufnahme durch aufbewahrte menschliche rote Blutzellen*

Fig. 14 zeigt die zeitabhängige Verminderung der Affinität von Erythrozyten zu Sauerstoff nach Inkubation mit IHP-beladenen V2-Vesikeln in 0,19-molarer IHP-Lösung bei pH 7,35. Der

Anstieg von $P_{O_2(1/2)}$ erreicht nach 4 Minuten seinen halben maximalen Wert. Die Kinetik der IHP-Aufnahme, gemessen als Anstieg von $P_{O_2(1/2)}$, hängt vom pH-Wert des Inkubationsmediums ab. In Fig. 15 ist die Halbwertzeit der IHP-Inkorportion gegen den pH-Wert aufgetragen. Die Aufnahme von IHP ist bei niedrigem pH-Wert (pH 7,3) eine langsamere Reaktion und bei höherem pH-Wert (pH 7,7) eine schnellere Reaktion. Die kürzere Halbwertzeit der Aufnahme bei hohem pH-Wert entspricht der größeren Menge des in die Erythrozyten inkorporierten IHP (siehe Fig. 6 und Fig. 13).

Die Kinetik der Inkorporierung von IHP wird durch die Anwesenheit von freiem IHP im äußeren Medium stark beeinflußt. Die Entfernung von freiem IHP durch Dialyse oder Gelfiltration der Vesikelsuspension führt zu einer Verlängerung der Halbwertzeit der IHP-Aufnahme auf 30 Minuten bei pH 7,4 (siehe Fig. 16).

*ATP-Konzentration in IHP-beladenen Erythrozyten*

Der Gehalt der Erythrozyten an Adenosintriphosphat (ATP) ist von großem Interesse vom Standpunkt der Konservierung von roten Blutzellen und der Erhaltung der einwandreien Funktion. Die ATP-Konzentration wurde in roten Blutzellen gemessen, in die leere V2-Vesikel und IHP-beladene V2-Vesikel unter den beschriebenen Bedingungen eingebaut worden waren. Alle Messungen wurden in isotonischem 0,1-molarem bis-Tris-Puffer von pH 7,4 durchgeführt. Die ATP-Konzentration wurde mit dem Luciferin-Luciferase-System gemessen:

Enzym + Luciferin + ATP ⇌ E—S—AMP + Pyrophosphat
(E) (S)

$O_2$

↓

E + oxydiertes Substrat + AMP + $CO_2$ + $h\nu$

Die Reaktion ist so wirksam, daß für jedes verwendete ATP-Molekül ein Proton gebildet wird. Der Einbau der leeren Vesikel oder der IHP-beladenen Vesikel ist ohne wesentlichen Einfluß auf die ATP-Konzentration in den Erythrozyten.

TABELLE 2

*ATP-Gehalt von Erythrozyten*

| | ATP [µm/ml RBC] |
|---|---|
| Erythrozyten | 0,92 ± 10% |
| Erythrozyten-V2 | 0,84 |
| Erythrozyten-V2-HIP | 0,93 |

Diese Werte gelten für RBC mit einem Alter von 1 Woche. Das Fehlen einer Veränderung der ATP-Konzentration im RBC nach der Aufnahme von IHP läßt unveränderte Lebensfähigkeit der Zellen, Funktionalität und Plastizität der RBC erkennen.

*$O_2$-Freisetzung durch Fusion von IHP-beladenen Vesikeln mit Erythrozyten*

Die "Rechtsverschiebung" der $O_2$-Bindungskurven nach der Aufnahme von IHP ist in Fig. 17 dargestellt. Nach Fusion von 41 Tage alten Erythrozyten, die in isotonischem bis-Tris-Puffer von pH 7,4 suspendiert waren, mit den IHP-beladenen V2-Vesikeln steigt der $O_2$-Halbsättigungsdruck von 7 auf 28 Torr. Dies bedeutet, daß die normalen, aber gealterten Erythrozyten bei 25°C bis zu 95% mit $O_2$ unter einem $O_2$-Partialdruck von 30 Torr beladen sind, während die IHP-beladenen Erythrozyten unter den gleichen Bedingungen nur zu 53% mit $O_2$ beladenes Hämoglobin enthalten. Etwa 60% des Hämoglobins in den Erythrozyten haben nach der Fusion der IHP-beladenen Vesikel mit den Erythrozyten IHP gebunden.

Unter physiologischen Bedingungen (bei 37°C) wird ein $O_2$-Halgsättigungsdruck von 60 Torr für IHP-beladene Erythrozyten bei pH 7,4 berechnet. Unter einem kritischen $O_2$-Partialdruck von 30 Torr im Gehirn würden 80% des Hämoglobins aus vesikelbehandelten Erythrozyten den gebundenen Sauerstoff abgeben, während normale, unbehandelte Erythrozyten unter diesen Bedingungen nur 20 bis 25% des Sauerstoffs abgeben würden. Die effektive Affinität der Erythrozyten kann zwischen diesen beiden Grenzen entweder durch Verändern der IHP-Konzentration in den Lipidvesikeln oder des Verhältnisses von behandelten zu unbehandelten Erythrozyten im Blut variiert werden.

Dieses Ergebnis zeigt, daß die erfindungsgemäß vorgeschlagene Methode des Einbaues von IHP in die Erythrozyten eine lang andauernde, starke und geregelte Verminderung der Affinität von Hämoglobin in intakten Zellen zu Sauerstoff ermöglicht. Die in dieser Weise mit IHP beladenen

Erythrozyten eignen sich besonders zur Regelung der Sauerstoffversorgung der Gewebe in den oben genannten Fällen.

*Anpassung von Ratten und Hunden an große Höhen*

Eine Ratte mit einem Körpergewicht von 200 g und einem Blutvolumen von 14 ml ($P_{O_{2(1/2)}}$ = 14,0 Torr bei 25°C und pH 7,4) wurde in einer Kammer unter sinkendem $O_2$-Partialdruck gehalten. Bei einem O-Partialdruck von 120 Torr entsprechend einer Höhe von 13200 m taumelte die Ratte als Folge von Sauerstoffmangel in den Muskeln der Extremitäten zu Boden. Der Sauerstoffdruck in der Kammer wurde dann schnell wieder auf den normalen Wert gebracht, worauf die Ratte sich normal verhielt. Aus diesem Tier wurde 1 ml Blut entnommen. Die Erythrozyten wurden isoliert und in der oben beschriebenen Weise mit IHP beladen. Die IHP-beladenen Erythrozyten wurden erneut im Plasma suspendiert ($P_{O_{2(1/2)}}$ = 28,0 Torr bei 25°C und pH 7,4) und dann in die Ratte rückübertragen. Nachdem nun der $O_2$-Partialdruck gesenkt worden war, taumelte die Ratte bei 100 mm Hg (etwa 14200 m Höhe) zu Boden. Diese Behandlung verursachte somit eine Anhebung der oberen Grenze der Höhe um +8%. Der Höhenanpassungsversuch wurde mit dieser Ratte 24 Stunden später wiederholt und führte zum gleichen Ergebnis.

Bei einem anderen Versuch wurde ein Hund mit einem Körpergewicht von 9 kg und einem Blutvolumen von 630 ml ($P_{O_{2(1/2)}}$ = 10,8 Torr bei 25°C und pH 7,4) in einer Kammer unter sinkendem $O_2$-Partialdruck gehalten. Bei einem $O_2$-Partialdruck von 140 Torr (= 12200 m Höhe) taumelte der Hund zu Boden. Dann wurde der $O_2$-Druck in der Kammer schnell wieder auf den normalen Wert gebracht, worauf der Hund sich normal verhielt. Diesem Hund wurden 100 ml Blut entnommen. Die Erythrozyten wurden in der unter "Methoden" beschriebenen Weise isoliert und mit IHP beladen. Die IHP-beladenen Erythrozyten wurden erneut in Serum suspendiert ($P_{O_{2(1/2)}}$ = 15,0 Torr bei 25°C und pH 7,4) und dann in den Hund rückübertragen. Nach der Senkung des $O_2$-Partialdrucks fiel der Hund bei 110 Torr (= 13800 m Höhe) zu Boden. Diese Behandlung verursachte somit eine Anhebung der oberen Grenze der Höhe um +13%. Diese obere Höhe wurde über 2 Tage mit dem gleichen Ergebnis gemessen.

Beide Tiere waren lebend und wohlauf, als sie zuletzt 4 Monate (Ratte) und 1 Monat (Hund) nach den Versuchen beobachtet wurden.

*Menschliche Erythrozyten mit eine Hämoglobinmutante von hoher $O_2$-Affinität*

Eine 19 Jahre alte Patientin mit einem Hämoglobinmutante von unbekannter Strucktur ($Hb_{Mainz}$) mit hoher Affinität zu Sauerstoff spendete Blut. Bei 25°C und pH 7,4 zeigten ihre frischen Erythrozyten einen $P_{O_{2(1/2)}}$ von 7,5 Torr. Die Sauerstoffzufuhr zu den Geweben war somit auf 50% verringert. Auf Grund des Sauerstoffmangels in ihren Geweben erhielt diese Patientin alle 6 Wochen Bluttransfusionen. Die Beladung dieser Erythrozyten mit IHP auf die unter "Methoden" beschriebene Weise hatte einen Anstieg von $P_{O_{2(1/2)}}$ auf 18,7 Torr zur Folge. Diese zeigt, daß die hohe Affinität des Bluts dieser Patientin zu Sauerstoff nach dem Verfahren gemäß der Erfindung auf Werte herabgesetzt werden kann, die über dem normalen Wert von frischen Erythrozyten liegen und einer Steigerung der Sauerstoffzufuhr von +23% eines vollständigen Blutaustausches entsprechen.

**Patentansprüche**

1. Modifizierte intakte Erythrozyten, die eine verbesserte Sauerstoffabgabe des Blutes gewährleisten, dadurch gekennzeichnet, daß in sie allosterische Effektoren mit Hilfe von Lipidvesikeln irreversibel inkorporiert sind und diese allosterischen Effektoren an das Hämoglobin in den Erythrozyten gebunden sind.

2. Modifizierte intakte Erythrozyten nach Anspruch 1, dadurch gekennzeichnet, daß der allosterische Effektor Inosithexaphosphat ist.

3. Modifizierte intakte Erythrozyten nach Anspruch 1, dadurch gekennzeichnet, daß der allosterische Effekt Inositpentaphosphat ist.

4. Modifizierte intakte Erythrozyten nach Ansprüchen 1—3, dadurch gekennzeichnet, daß die Lipidvesikel Gemische von Phosphatidylcholin/Phosphatidylserin/Cholesterin im Molverhältnis von 10 bis 5:4 bis 1:10 bis 3 sind.

5. Modifizierte intakte Erythrozyten nach Ansprüchen 1, 2 und 4, dadurch gekennzeichnet, daß der allosterische Effektor Inosithexaphosphat und die Lipidvesikel ein Gemisch von Phosphatidylcholin/Phosphatidylserin/Cholesterin im Molverhältnis von 8:2:7 sind.

6. Extrakorporales Blut oder Blutkonserven, dadurch gekennzeichnet, daß sie modifizierte intakte Erythrozyten nach Ansprüchen 1—5 enthalten.

7. Verfahren zur Herstellung von modifizierten intakten Erythrozyten nach Ansprüchen 1 bis 5, dadurch gekennzeichnet, daß man mit allosterischen Effektoren beladene Lipidvesikel in Trägerfluiden in das Innere von Erythrozyten irreversibel inkorporiert und die allosterischen Effektoren an das Hämoglobin in den Erythrozyten bindet.

8. Verfahren nach Anspruch 7, dadurch gekennzeichnet, daß man

a) Inosithexaphosphat in einer isotonischen Pufferlösung löst, bis die Lösung gesättigt ist,
b) in dieser Lösung ein Lipidgemisch suspendiert, das Phosphatidylcholin, Phosphatidylserin und Cholesterin im Molverhältnis von 10 bis 5:4 bis 1:10 bis 3 enthält,
c) die erhaltene Suspension der Desintegration mit Ultraschall oder einem Einspritzverfahren unterwirft,
d) das Gemisch zentrifugiert und hierdurch die überstehende Suspension abtrennt, die die mit Inosithexaphosphat angereicherten kleinen Lipidvesikel sowie freies Inosithexaphosphat enthält,
e) worauf man menschliche Erythrozyten, die vorher vom Blutplasma durch Zentrifugieren abgetrennt worden sind in der überstehenden Suspension suspendiert, die die mit Inosithexaphosphat angereicherten kleinen Lipidvesikel sowie freies Inosithexaphosphat enthält,
f) die erhaltene Suspension inkubiert und hierdurch die Vesikel mit den Erythrozyten fusioniert, und
g) die nunmehr modifizierten intakten Erythrozyten mit isotonischer Kochsalzlösung oder isotonischem Puffer wäscht und quantitativ von außen vorhandenem freiem Inosithexaphosphat befreit und im Blutplasma bzw. Blutplasma-Ersatzlösungen suspendiert.

9. Zusammensetzungen zur Steigerung der Sauerstoffabgabe des Blutes, dadurch gekennzeichnet, daß sie allosterische Effektoren und Gemische von Phosphatidylcholin, Phosphatidylserin und Cholesterin sowie unbedenkliche Träger- und Hilfsstoffe enthalten.

10. Zusammensetzung nach Anspruch 9, dadurch gekennzeichnet, daß der allosterische Effektor Inosithexaphosphat ist.

11. Zusammensetzung nach Ansprüchen 9 und 10, dadurch gekennzeichnet, daß die Lipidvesikel Gemische von Phosphatidylcholin, Phosphatidylserin und Cholesterin im Molverhältnis von 10 bis 5:4 bis 1:10 bis 3 sind.

12. Zusammensetzungen nach Ansprüchen 9—11, dadurch gekennzeichnet, daß sie Inosithexaphosphat und ein Gemisch von Phosphatidylcholin, Phosphatidylserin und Cholesterin im Molverhältnis von 8:2:7 enthalten.

**Revendications**

1. Erythrocytes intacts modifiés, qui assurent une meilleure libération de l'oxygène par le sang, caractérisés en ce que, de facon irréversible, on leur incorpore des effecteurs allostériques à l'aide de vésicules de lipides et que ces effecteurs allostériques sont fixés sur l'hémoglobin dans les érythrocytes.

2. Erythrocytes intacts modifiés selon la revendication 1, caractérisés en ce que l'effecteur allostérique est l'hexaphosphate d'inositol.

3. Erythrocytes. intacts modifiés selon la revendication 1, caractérisés en ce que l'affecteur allostérique est le pentaphosphate d'inositol.

4. Erythrocytes intacts modifiés selon l'une quelconque des revendications 1 à 3, caractérisés en ce que les vésicules de lipides sont des mélanges de phosphatidylcholine/phosphatidylsérine/cholestérine dans un rapport molaire de 10 à 5:4 à 1:10 à 3.

5. Erythrocytes intacts modifiés selon l'une quelconque des revendications 1, 2 et 4, caractérisés en ce que l'effecteur allostérique est l'hexaphosphate d'inositol et que les vésicules de lipides sont un mélange de phosphatidylcholine/phosphatidylsérine/cholestérine dans un rapport molaire de 8:2:7.

6. Sang extracorporel ou conserves de sang, caractérisés en ce qu'ils contiennent des érythrocytes intacts modifiés selon l'une quelconque des revendications 1 à 5.

7. Procéde d'obtention d'érythrocytes modifiés selon l'une quelconque des revendications 1 à 5, caractérisé en ce qu'on incorpore de façon irréversible dans l'intérieur des érythrocytes, des vésicules de lipides chargées d'effecteurs allostériques dans des véhicules liquides et qu'on fixe les effecteurs allostériques sur l'hémoglobine dans les érythrocytes.

8. Procédé selon la revendication 7, caractérisé en ce qu'il consiste à:
a) dissoudre l'hexaphosphate d'inositol dans une solution tampon isotonique, jusqu'à saturation de la solution,
b) mettre un mélange de lipides en suspension dans cette solution, lequel mélange contient de la phosphatidylcholine, de la phosphatidylsérine et de la cholestérine dans un rapport molaire de 10 à 5:4 à 1:10 à 3,
c) soumettre la suspension obtenue à une désintégration par ultra-sons ou par pulvérisation,
d) centrifuger le mélange et séparer ainsi la suspension surnageante qui contient les petites vésicules de lipides enrichies en hexaphosphate d'inositol, ainsi que de l'hexaphosphate d'inositol libre,
e) placer ensuite des érythrocytes humains qui ont été au préalable séparés du plasma sanguin par centrifugation, dans la suspension qui contient les petites vésicules de lipides enrichies en hexaphosphate d'inositol ainsi que de l'hexaphosphate d'inositol libre,
f) incuber la suspension obtenue et ainsi faire fusionner les vésicules avec les érythrocytes, et
g) laver les érythrocytes intacts maintenant modifiés avec un solution isotonique de chlorure de

sodium ou un tampon isotonique, et libérer quantitativement l'hexaphosphate d'inositol venant de l'extérieur, puis mettre en suspension dans du plasma sanguin ou dans des solutions de plasma sanguin synthétiques.

9. Compositions pour augmenter la libération de l'oxygène du sang, caractérisés en ce qu'elles contiennent des effecteurs allostériques et des mélanges de phosphatidylcholine, de phosphatidylsérine et de cholestérine, ainsi que des véhicules et des additifs acceptables.

10. Compositions selon la revendication 9, caractérisés en ce que l'effectuer allostérique est l'hexaphosphate d'inositol.

11. Compositions selon l'une quelconque des revendications 9 ou 10, caractérisées en ce que les vésicules de lipides sont des mélanges de phosphatidylcholine, de phosphatidylsérine et de cholestérine dans un rapport molaire de 10 à 5:4 à 1:10 à 3.

12. Compositions selon l'une quelconque des revendications 9 à 11, caractérisées en ce qu'elles contiennent de l'hexaphosphate d'inositol et un mélange de phosphatidylcholine, de phosphatidylsérine et de cholestérine dans un rapport molaire de 8:2:7.

## Claims

1. Modified intact erythrocytes ensuring improved oxygen release of the blood, characterized in that allosteric effectors are irreversibly incorporated into the erythrocytes by means of lipid vesicles and these allosteric effectors are bound to the haemoglobin in the erythrocytes.

2. Modified intact erythrocytes according to claim 1, characterized in that said allosteric effector is inositol hexaphosphate.

3. Modified intact erythrocytes according to claim 1, characterized in that said allosteric effector is inositol pentaphosphate.

4. Modified intact erythrocytes according to claims 1 to 3, characterized in that said lipid vesicles are mixtures of phosphatidylcholine/phosphatidylserine/cholesterol in a molar ratio of 10 to 5.4 to 1:10 to 3.

5. Modified intact erythrocytes according to claims 1, 2 and 4, characterized in that said allosteric effector is inositol hexaphosphate and said lipid vesicles are a mixture of phosphatidylcholine/phosphatidylserine/cholesterol in a molar ratio of 8:2:7.

6. Extracorporal blood or stored blood, characterized in that it contains modified intact erythrocytes according to claims 1 to 5.

7. A process for the preparation of modified intact erythrocytes according to claims 1 to 5, characterized in that lipid vesicles loaded with allosteric effectors in carrier fluids are irreversibly incorporated into the interior of erythrocytes and the allosteric effectors are bound to the haemoglobin in the erythrocytes.

8. A process according to claim 7, characterized by

(a) dissolving inositol hexaphosphate in an isotonic buffer solution until the solution is saturated,

(b) suspending in this solution a lipid mixture containing phosphatidylcholine, phosphatidylserine and cholesterol in the molar ratio 10 to 5:4 to 1:10 to 3,

(c) subjecting the resulting suspension to disintegration with ultrasonic waves or an injection process,

(d) centrifuging the mixture to separate the supernatent suspension which contains the small inositol hexaphosphate enriched lipid vesicles and free inositol hexaphosphate, thereafter,

(e) suspending human erythrocytes having previously been separated from the blood plasma by centrifugation in the supernatent suspension which contains the small inositol hexaphosphate-enriched lipid vesicles and free inositol hexaphosphate,

(f) incubating the resulting suspension thereby effecting fusion of the vesicles with the erythrocytes and

(g) washing the now modified intact erythrocytes with isotonic NaCl solution or isotonic buffer and quantitatively freeing them from free inositol hexaphosphate being outside the erythrocytes and suspending them in blood plasma or blood plasma substitute solutions.

9. Compositions for increasing the oxygen release of blood, characterized in that they contain allosteric effectors and mixtures of phosphatidylcholine, phosphatidylserine and cholesterol and acceptable carrier materials and adjuvants.

10. Compositions according to claim 9, characterized in that said allosteric effector is inositol hexaphosphate.

11. Composition according to claims 9 and 10, characterized in that said lipid vesicles are mixtures of phosphatidylcholine, phosphatidylserin and cholesterol in a molar ratio of 10 to 5:4 to 1:10 to 3.

12. Compositions according to claims 9 to 11, characterized in that they contain inositol hexaphosphate and a mixture of phosphatidylcholine, phosphatidylserine and cholesterol in a molar ratio of 8:2:7.

FIG. 1

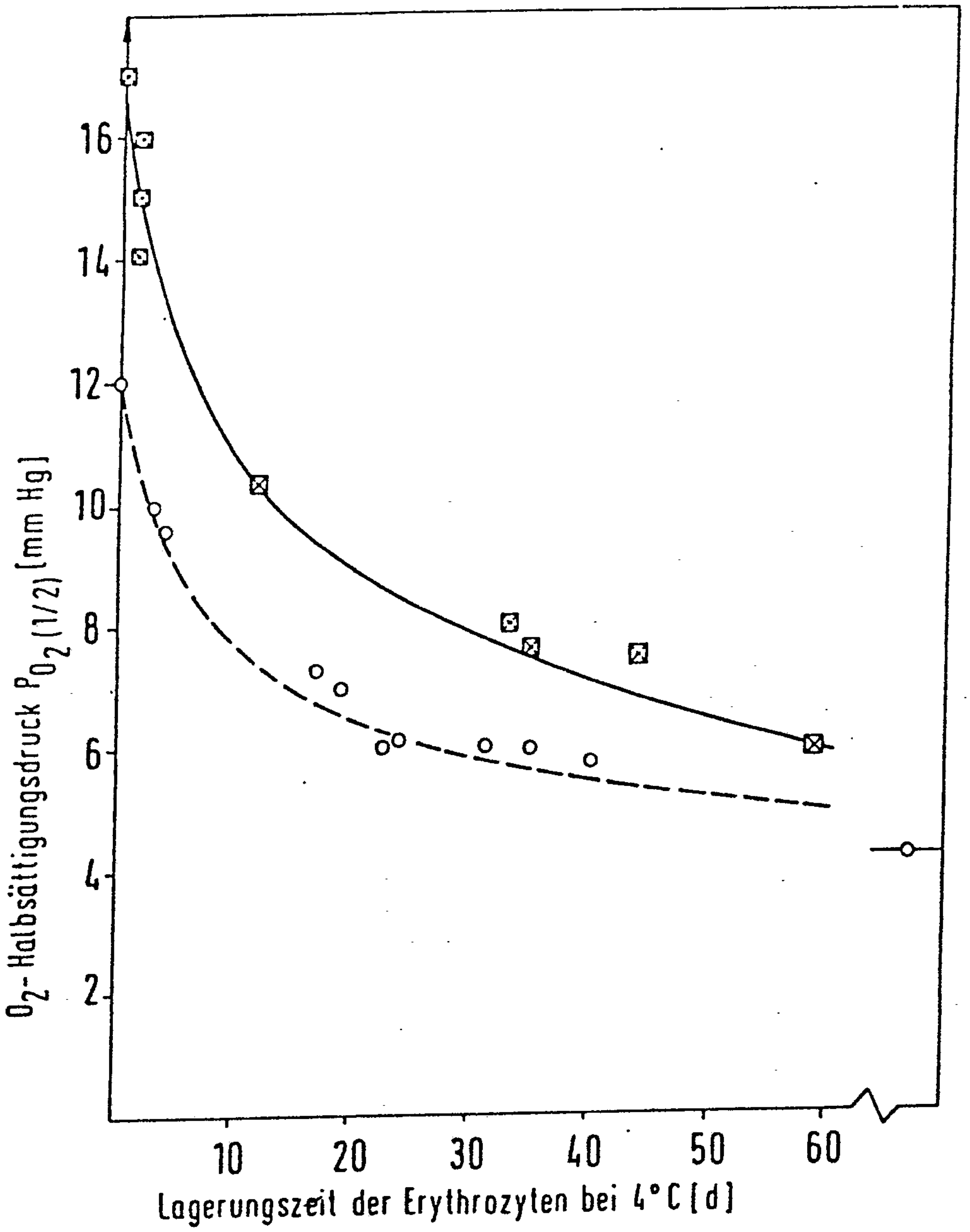

O2- Halbsättigungsdruck PO2(1/2) [mm Hg]
16
14
12
10
8
6
4
2
10
20
30
40
50
60
Lagerungszeit der Erythrozyten bei 4°C [d]

0 001 104

FIG. 2

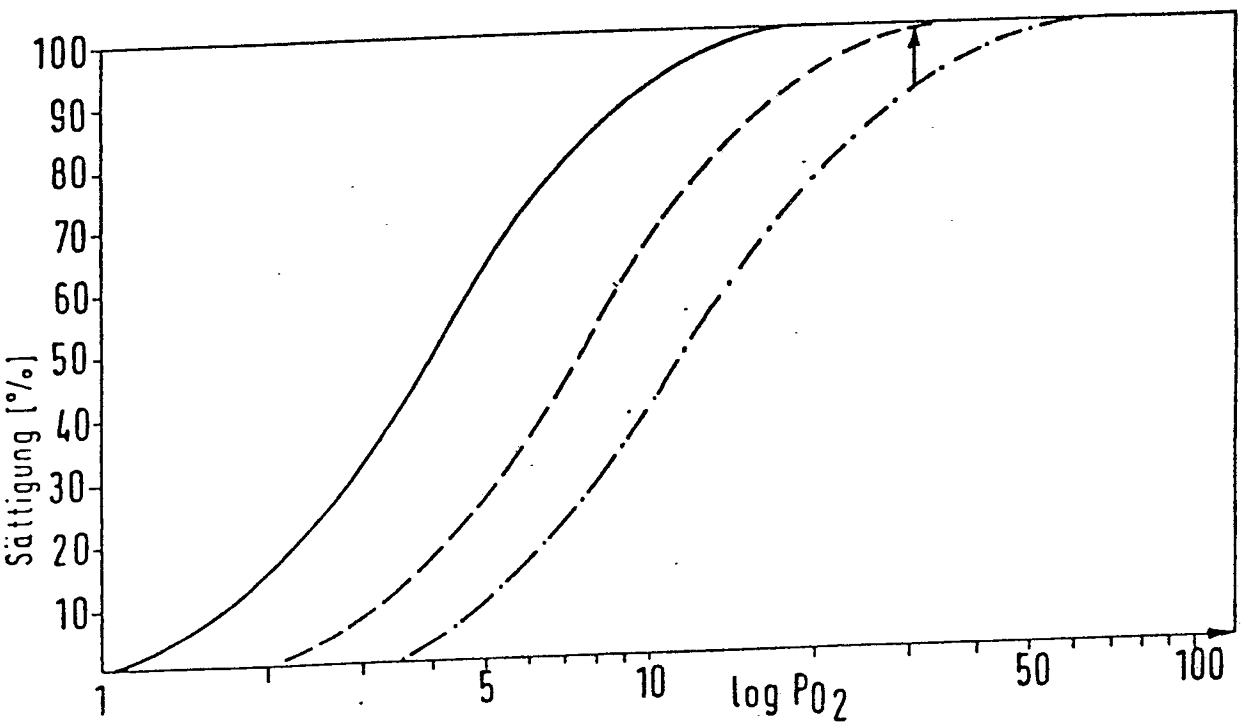


FIG. 3

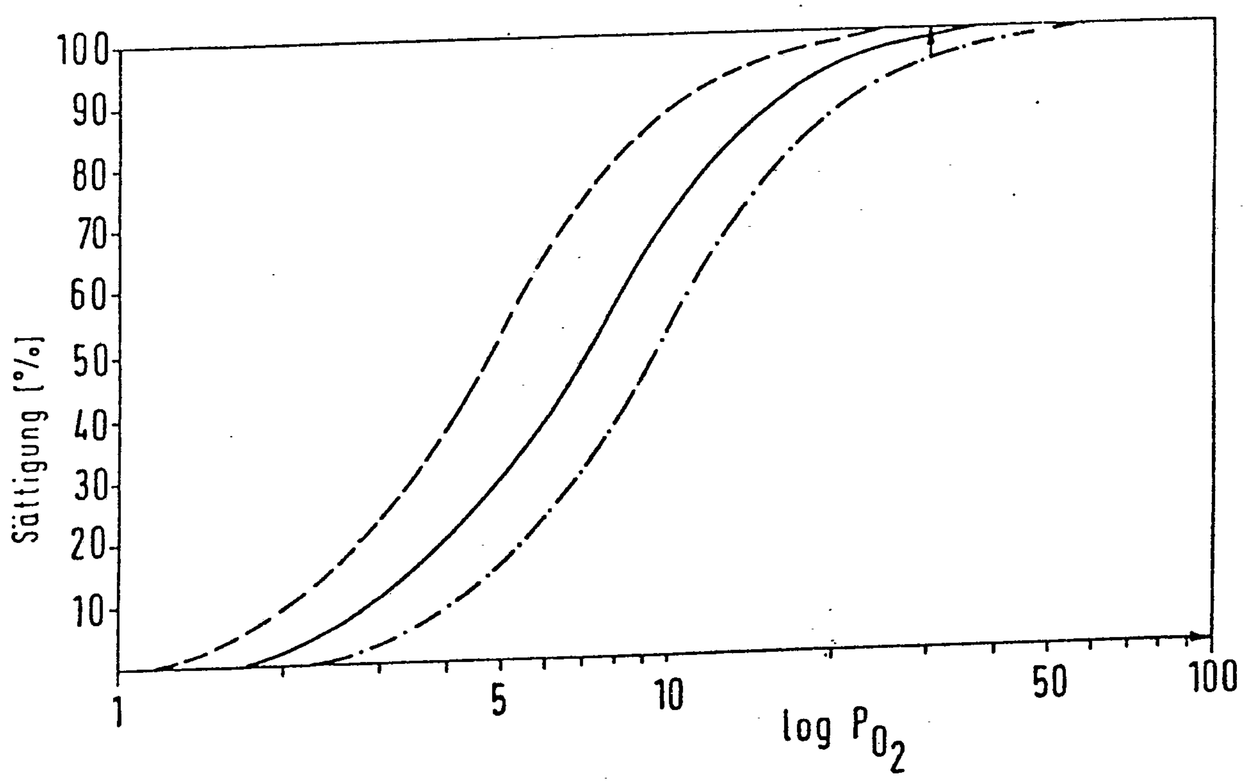

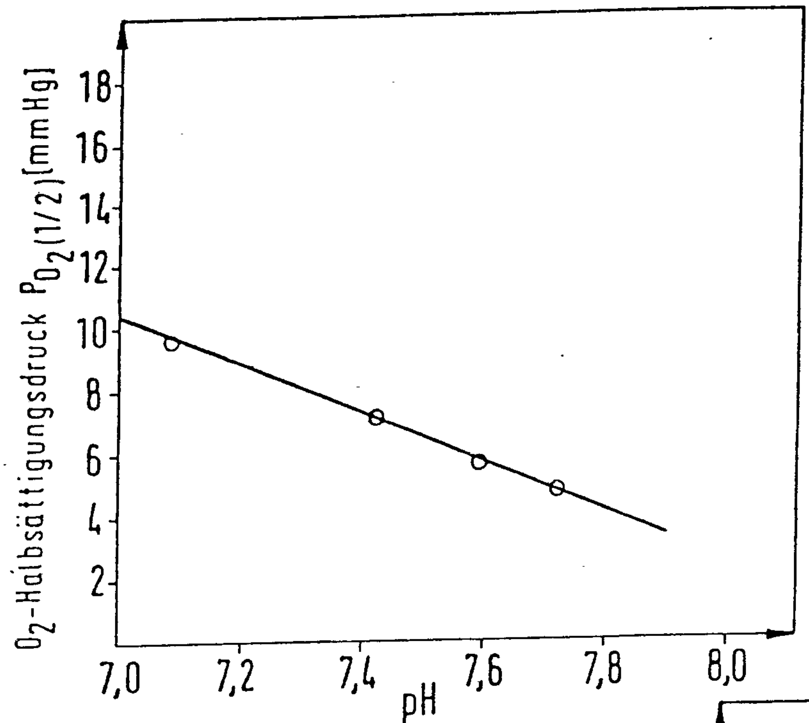
O2-Halbsättigungsdruck PO2(1/2) [mmHg]
18
16
14
12
10
8
6
4
2
7,0
7,2
7,4
7,6
7,8
8,0
pH

FIG. 4

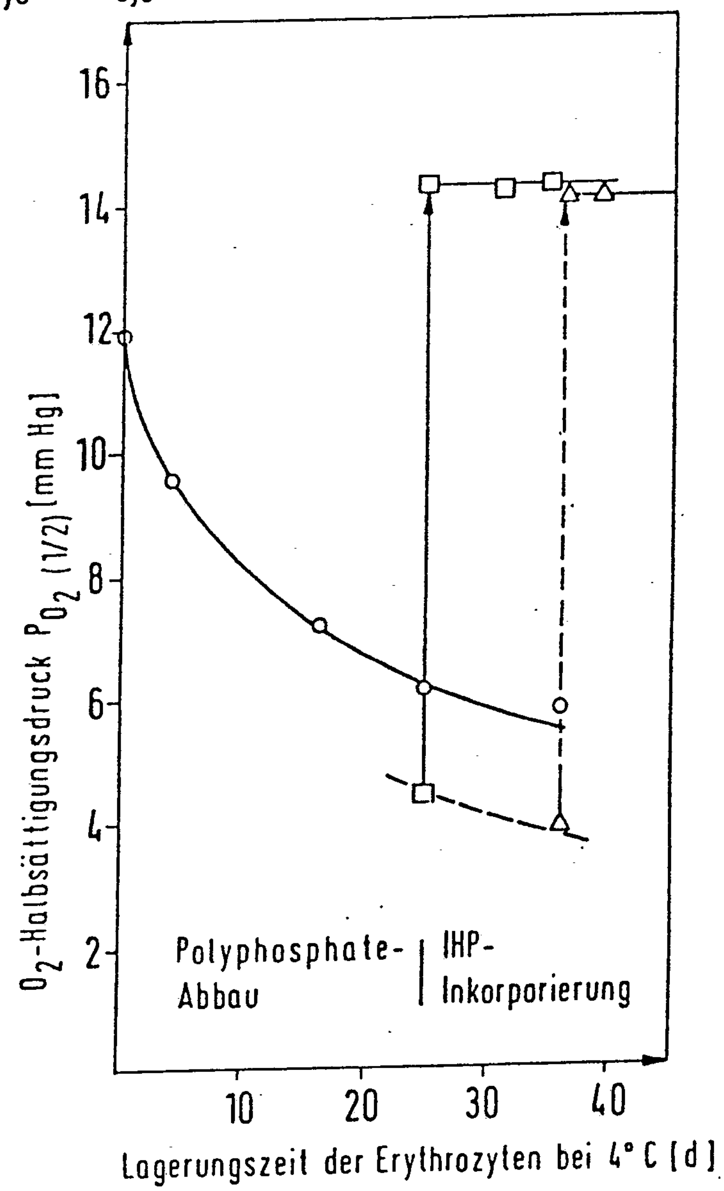
O2-Halbsättigungsdruck PO2 (1/2) [mm Hg]
16
14
12
10
8
6
4
2
Polyphosphate-Abbau
IHP-Inkorporierung
10
20
30
40
Lagerungszeit der Erythrozyten bei 4° C [d]

FIG. 5

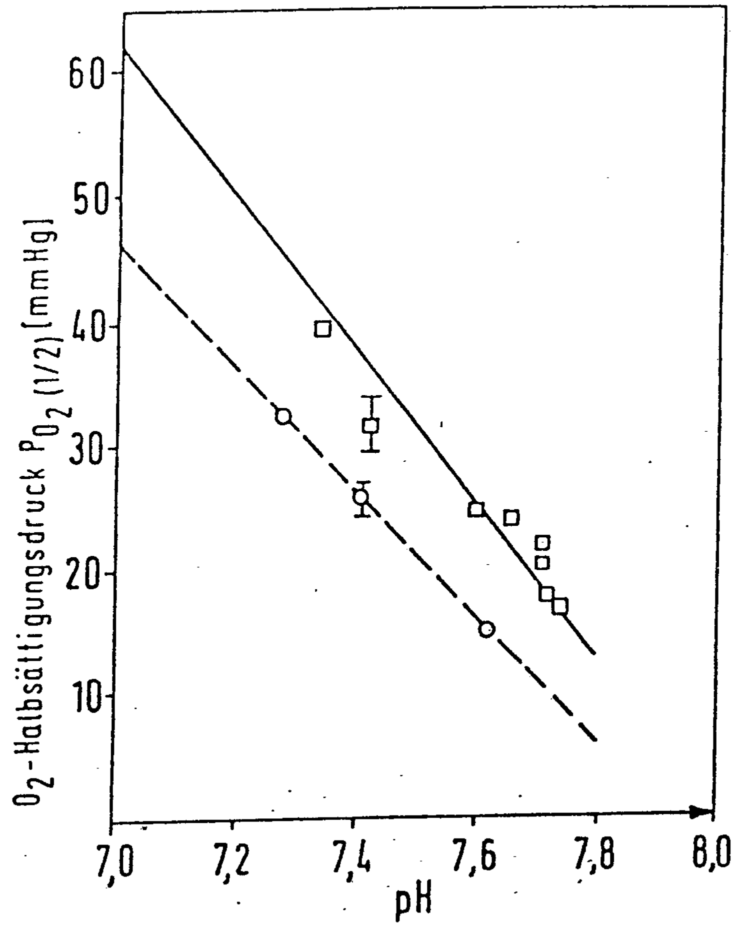
60
50
40
30
20
10
$O_2$-Halbsättigungsdruck $P_{O_2}$ (1/2) [mmHg]
7,0
7,2
7,4
7,6
7,8
8,0
pH

FIG. 6

FIG. 7

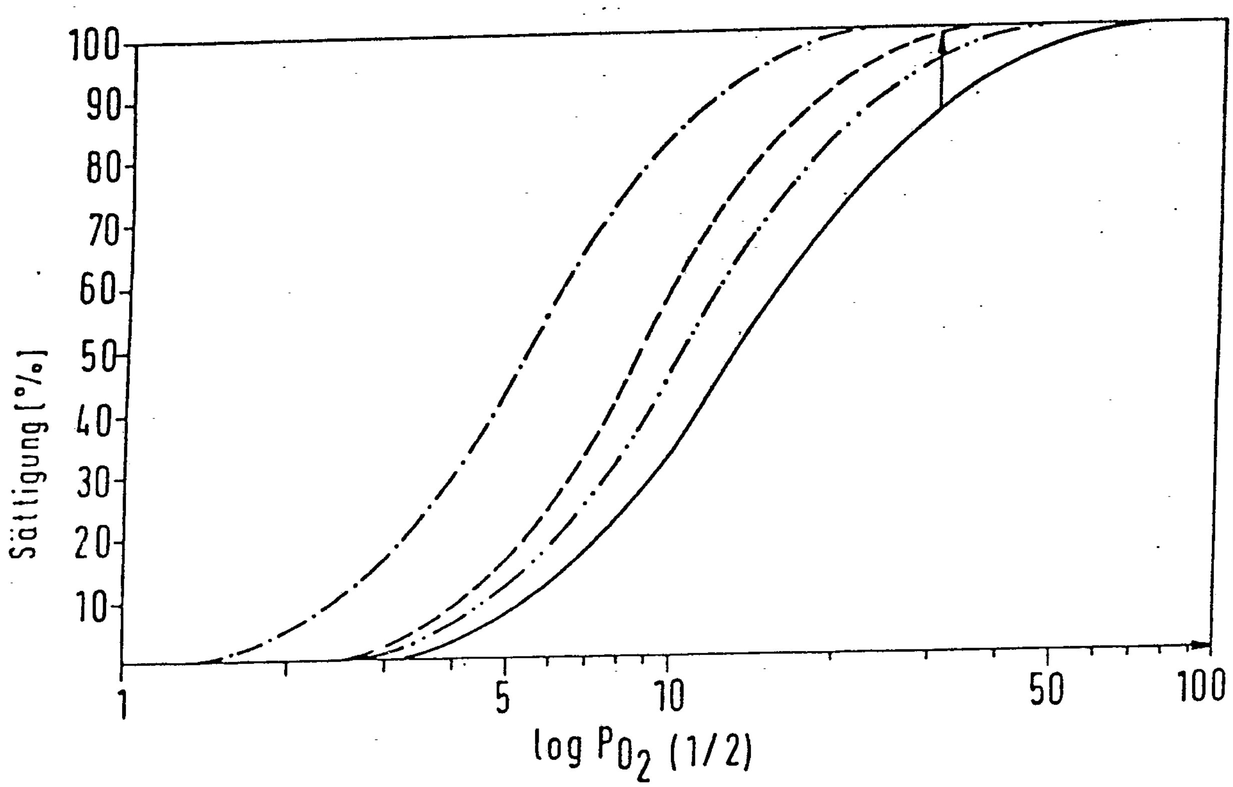
100
90
80
70
60
50
40
30
20
10
Sättigung [%]
1
5
10
50
100
log $P_{O_2}$ (1/2)

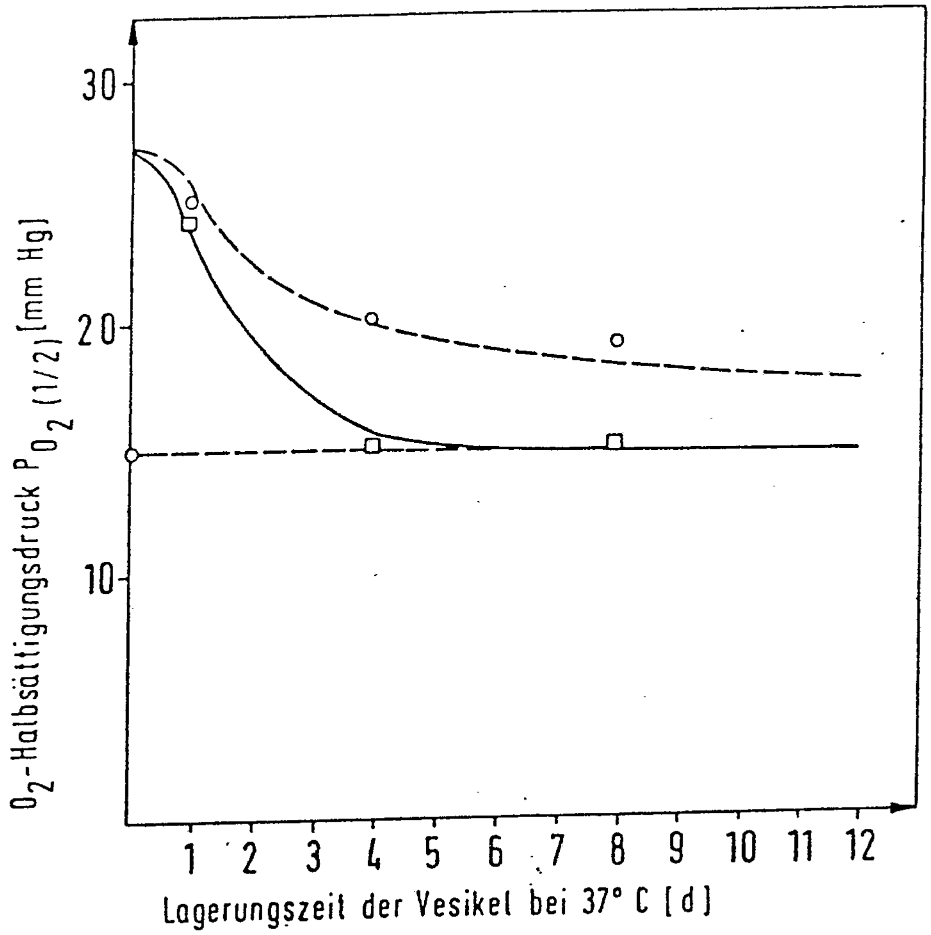
30
20
10
$O_2$-Halbsättigungsdruck $P_{O_2}$ (1/2) [mm Hg]
1 2 3 4 5 6 7 8 9 10 11 12
Lagerungszeit der Vesikel bei 37° C [d]

FIG. 8

FIG. 9

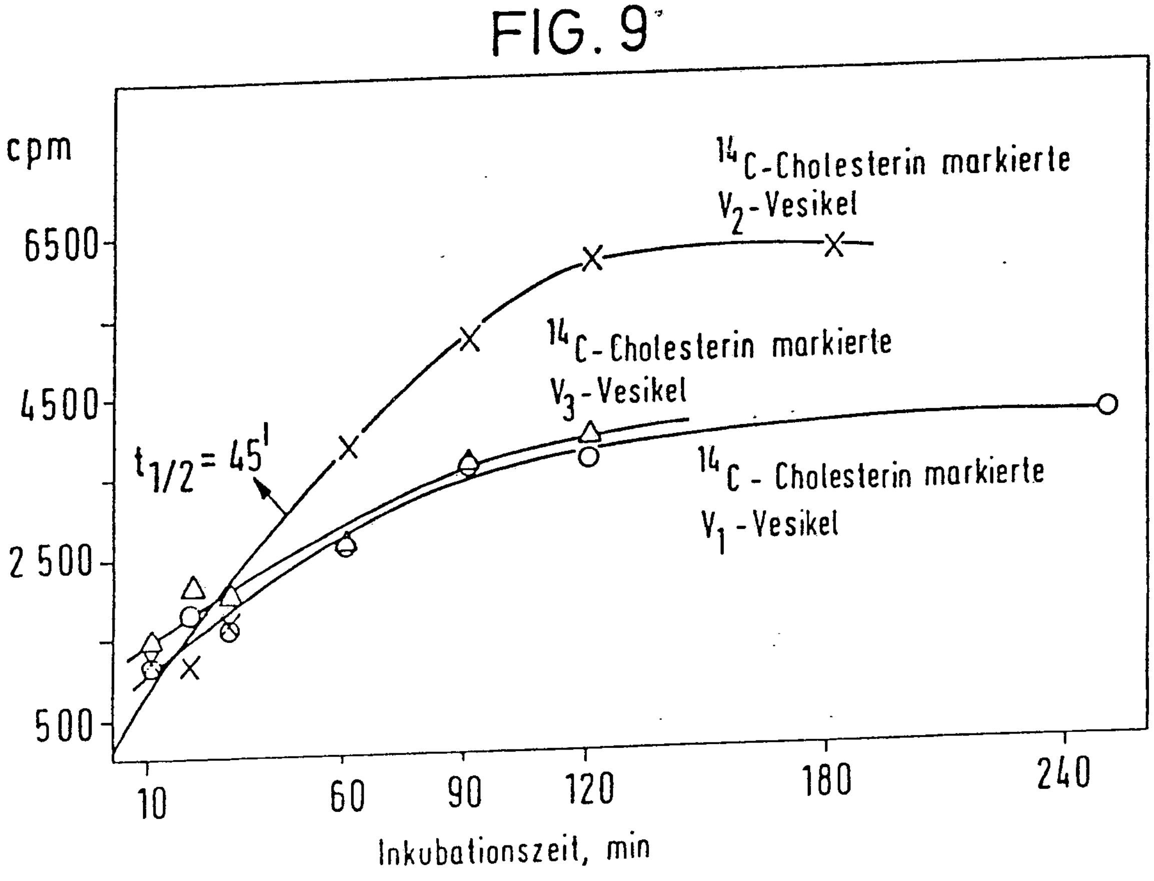
cpm
6500
4500
2500
500
$^{14}C$-Cholesterin markierte $V_2$-Vesikel
$^{14}C$-Cholesterin markierte $V_3$-Vesikel
$^{14}C$ - Cholesterin markierte $V_1$ - Vesikel
$t_{1/2} = 45'$
10 60 90 120 180 240
Inkubationszeit, min

FIG. 10

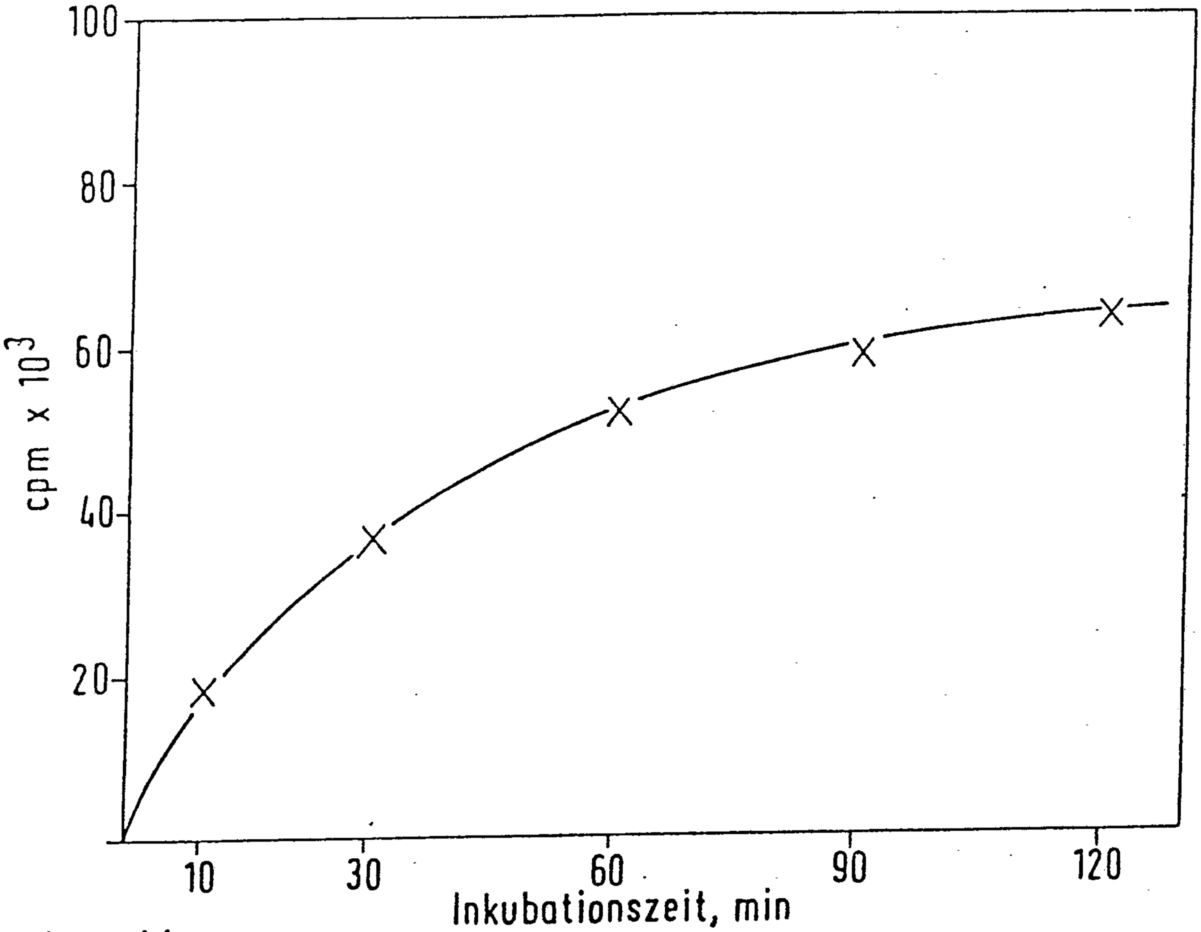
100
80
60
40
20
cpm x $10^3$
10
30
60
90
120
Inkubationszeit, min

FIG. 11

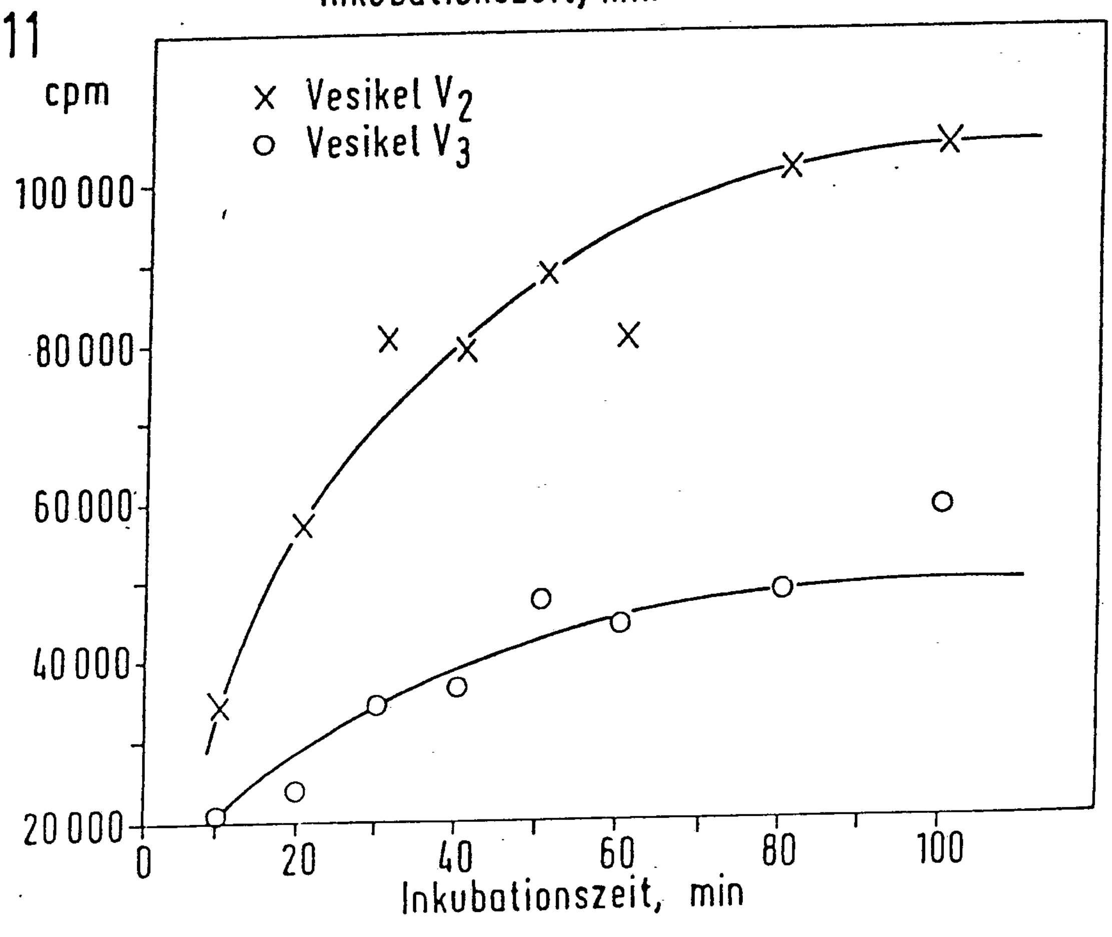
cpm
× Vesikel $V_2$
○ Vesikel $V_3$
100 000
80 000
60 000
40 000
20 000
0
20
40
60
80
100
Inkubationszeit, min

FIG. 12

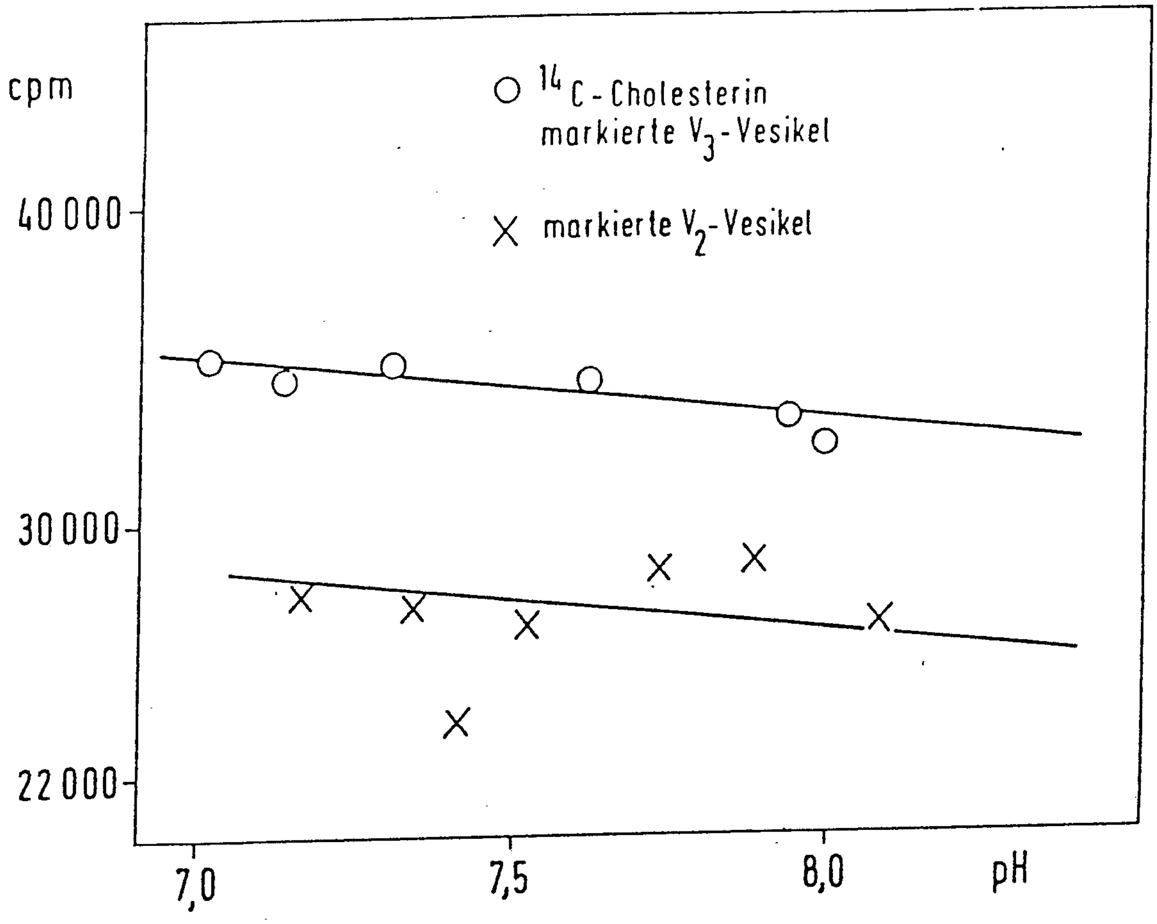
cpm
40 000
30 000
22 000
○ 14C-Cholesterin markierte V3-Vesikel
X markierte V2-Vesikel
7,0
7,5
8,0
pH

FIG. 13

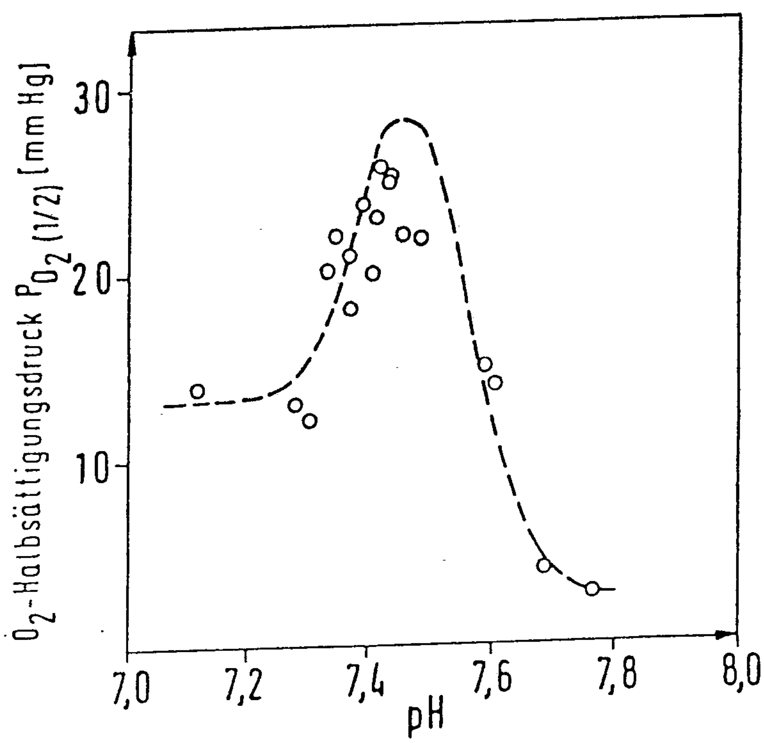
O2-Halbsättigungsdruck PO2 (1/2) [mm Hg]
30
20
10
7,0
7,2
7,4
7,6
7,8
8,0
pH

FIG. 14

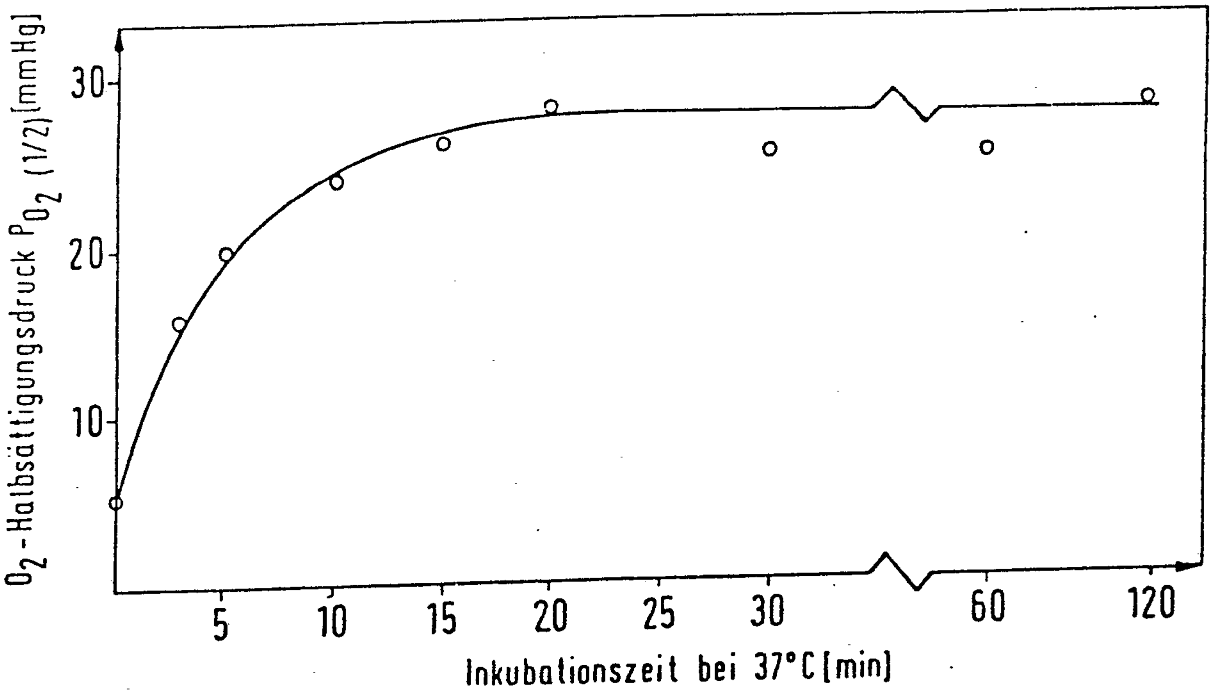
$O_2$-Halbsättigungsdruck $P_{O_2}$ (1/2) [mmHg]
30
20
10
5
10
15
20
25
30
60
120
Inkubationszeit bei 37°C [min]

FIG. 15

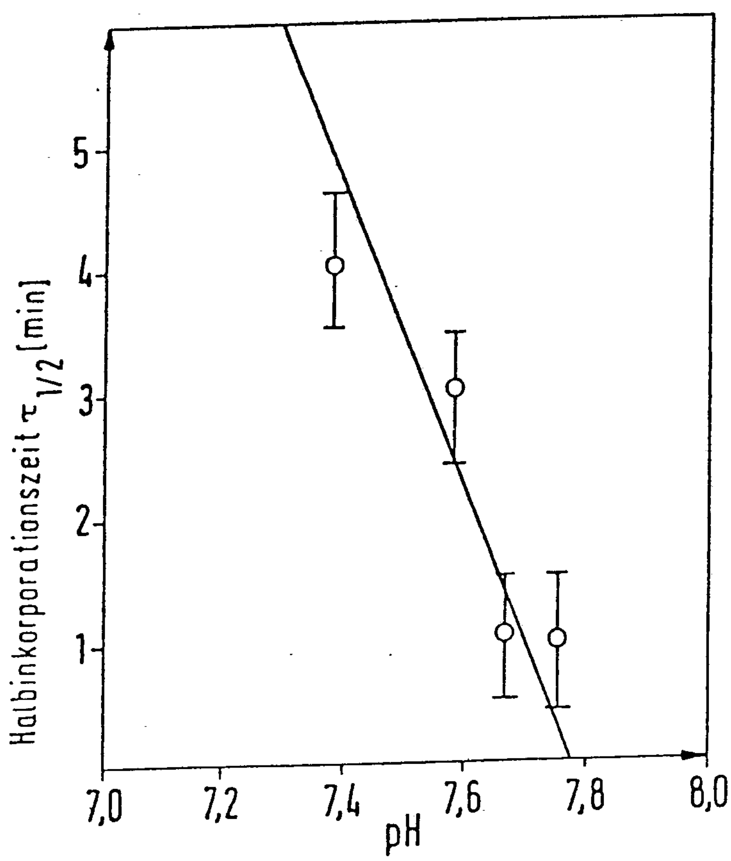
Halbinkorporationszeit $\tau_{1/2}$ [min]
5
4
3
2
1
7,0
7,2
7,4
7,6
7,8
8,0
pH

FIG. 16

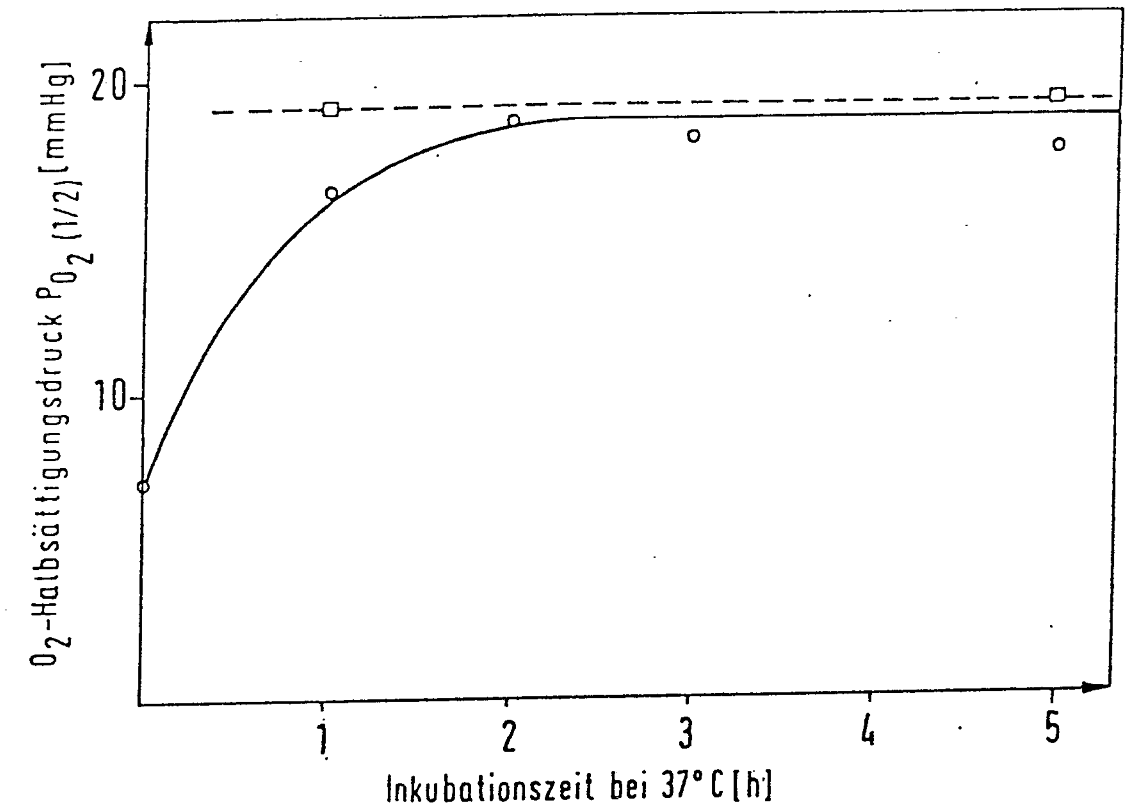
20
10
O2-Halbsättigungsdruck PO2 (1/2) [mmHg]
1
2
3
4
5
Inkubationszeit bei 37° C [h]

FIG. 17

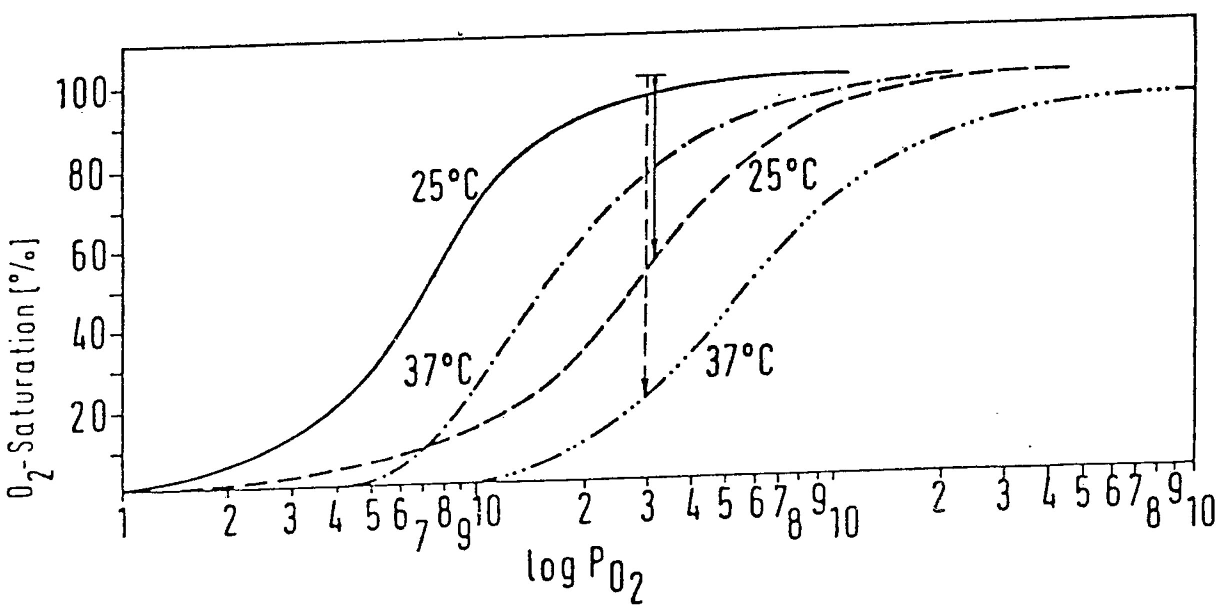
100
80
60
40
20
O2-Saturation [%]
25°C
37°C
25°C
37°C
1 2 3 4 5 6 7 8 9 10 2 3 4 5 6 7 8 9 10 2 3 4 5 6 7 8 9 10
log PO2

FIG. 16

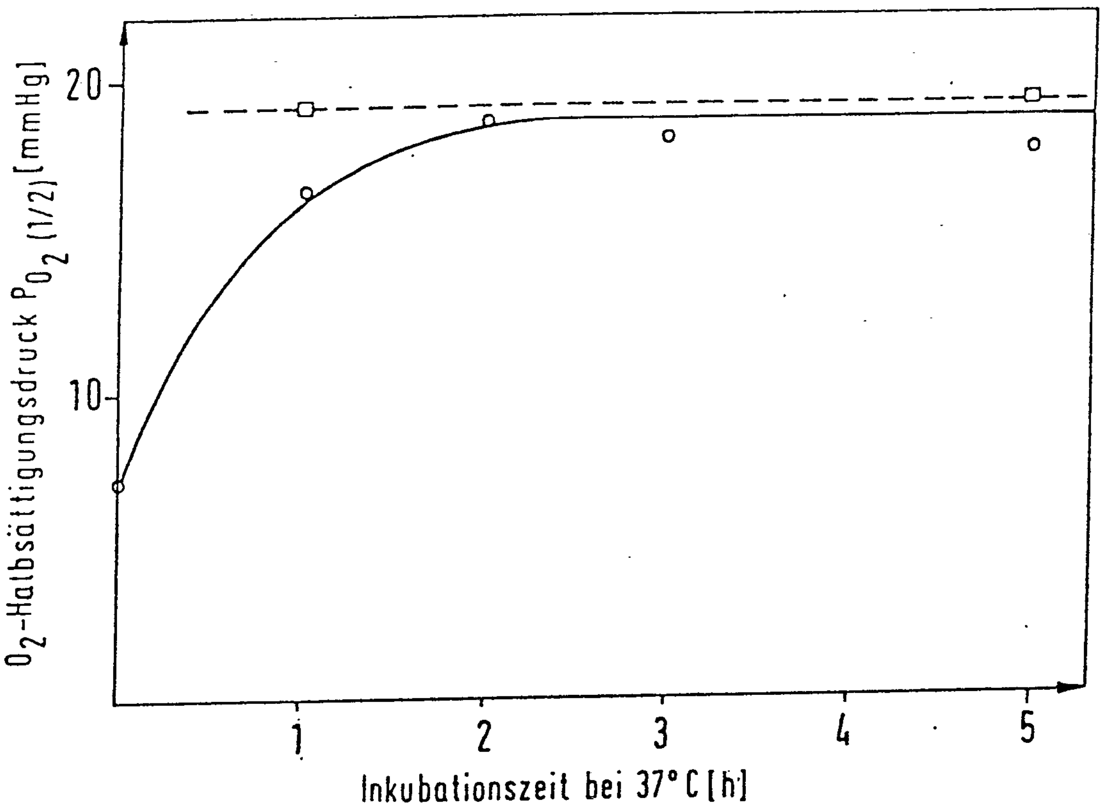
$O_2$-Halbsättigungsdruck $P_{O_2}$ (1/2) [mmHg]
20
10
1
2
3
4
5
Inkubationszeit bei 37° C [h]

FIG. 17

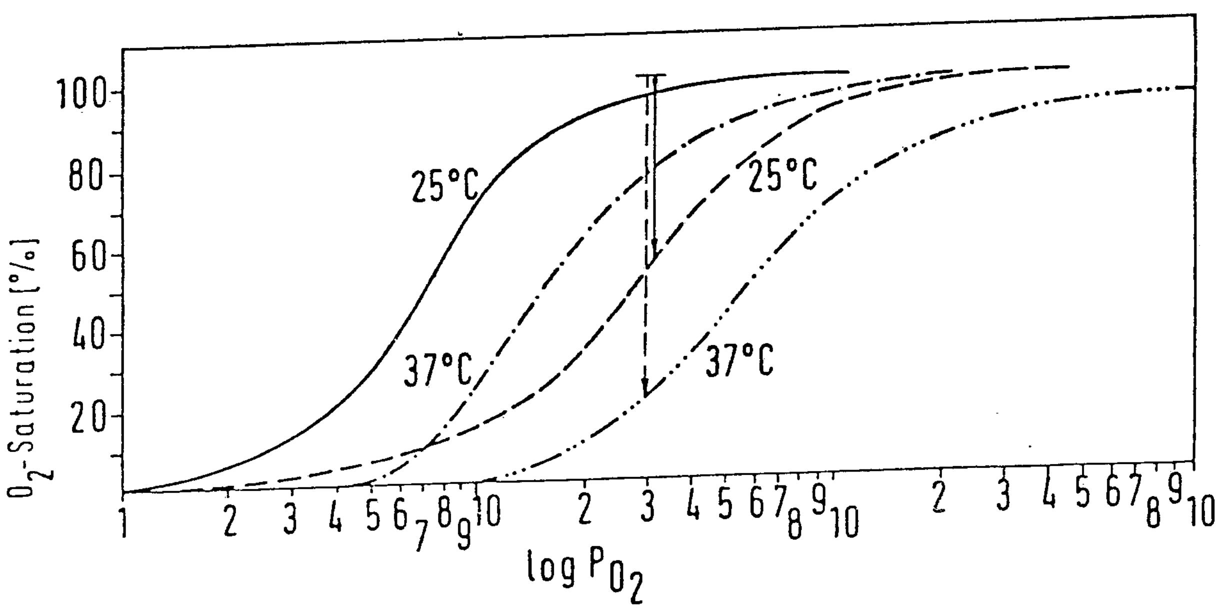
$O_2$-Saturation [%]
100
80
60
40
20
25°C
37°C
25°C
37°C
1 2 3 4 5 6 7 8 9 10 2 3 4 5 6 7 8 9 10 2 3 4 5 6 7 8 9 10
log $P_{O_2}$